(19) Europäisches Patentamt / European Patent Office / Office européen des brevets

(11) **EP 2 074 992 B1**

(12) **EUROPEAN PATENT SPECIFICATION**

(45) Date of publication and mention
of the grant of the patent:
**20.05.2015 Bulletin 2015/21**

(21) Application number: **09156199.3**

(22) Date of filing: **07.04.2006**

(51) Int Cl.:
*A61K 9/16* *(2006.01)*    *A61K 9/48* *(2006.01)*
*A61K 9/20* *(2006.01)*    *A61K 9/28* *(2006.01)*
*A61K 9/50* *(2006.01)*    *A61K 31/192* *(2006.01)*

(54) **Oral pharmaceutical formulations comprising salts of fenofibric acid**

Orale Pharmazeutische Formulierungen enthaltend Salze von Fenofibrinsäure

Formulations pharmaceutiques orales contenant des sels de l'acide fénofibrique

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(30) Priority: **08.04.2005 US 669699 P**

(43) Date of publication of application:
**01.07.2009 Bulletin 2009/27**

(62) Document number(s) of the earlier application(s) in
accordance with Art. 76 EPC:
**06799902.9 / 1 868 587**

(73) Proprietor: **ABBOTT LABORATORIES**
**Abbott Park, IL 60064-3500 (US)**

(72) Inventors:
• **Ju, Tzuchi, R.**
 **VERNON HILLS, IL 60061 (US)**
• **Davila, Claudia, M.**
 **SKOKIE, IL 60077 (US)**

• **Engh, Kevin, R.**
 **KENOSHA, WI, 53143 (US)**
• **Gao, Yi**
 **VERNON HILLS, IL 60061 (US)**
• **Gustavson, Linda, E.**
 **EVANSTON, IL 60203 (US)**
• **Jayaraman, Shyamala, C.**
 **GURNEE, IL 60031 (US)**
• **Leblond, David**
 **WADSWORTH, IL 60083 (US)**
• **Lee, Dennis, Y.**
 **HIGHLAND PARK, IL 60035 (US)**

(74) Representative: **Hubert, Philippe et al**
**Cabinet Beau de Loménie**
**158, rue de l'Université**
**75340 Paris Cedex 07 (FR)**

(56) References cited:
**WO-A-2004/054568     US-A1- 2005 148 594**

Remarks:
The file contains technical information submitted after
the application was filed and not included in this
specification

**Description**

<u>Field of the Invention</u>

The present invention relates to solid dosage forms comprising at least one of 2-[4-(4-chlorobenzoyl)phenoxy]-2-methyl-propanoic acid, salts of 2-[4-(4-chlorobenzoyl)phenoxy]-2-methyl-propanoic acid or buffered 2-[4-(4-chlorobenzoyl)phenoxy]-2-methyl-propanoic acid.

<u>Background of the Invention</u>

2-[4-(4-chlorobenzoyl)phenoxy]-2-methyl-propanoic acid,1-methylethyl ester, also known as "fenofibrate", from the family of fibrates, is a lipid-regulating agent. Fenofibrate is described in, for example, U.S. Patent Nos. 3,907,792, 4,895,726, 6,074,670 and 6,277,405. Fenofibrate is commercially available in a variety of different formulations and is used in the treatment of adult endogenous hyperlipidemias, hypercholesterolemias and hypertriglyceridemias. The active metabolite of fenofibrate is 2-[4-(4-chlorobenzoyl)phenoxy]-2-methyl-propanoic acid, which is also known as fenofibric acid.

One of the challenges associated with fibrates, such as fenofibrate, is that these compounds are hydrophobic and poorly soluble in water. Thus, the bioavailability of these compounds (i.e., their absorption in the digestive tract) can be low. Due to the hydrophobic nature and poor solubility of fenofibrate in water, absorption of fenofibrate in the digestive tract of a subject is increased after ingestion of food by the subject (when compared to when the subject ingests the fenofibrate under fasting conditions). This food effect is undesirable when comparing the bioavailability of fenofibrate in fed versus fasting conditions. Additionally, subject compliance is an issue with drugs having a food effect because the patient must coordinate administration of the drug with the ingestion of food. Recently, complex technologies have been used to overcome the food effect issues associated with fenofibrate.

In contrast to fenofibrate, fenofibric acid has higher solubility in the small intestine region. However, this enhanced solubility could cause problems in connection with controlling the delivery of fenofibric acid, salts of fenofibric acid or buffered fenofibric acid (such as, the potential for the Cmax to exceed the accepted (approved) limits of a reference pharmaceutical composition containing fenofibrate). For example, immediate release dosage forms comprising amorphous fenofibric acid are described, for example, in U.S. Patent Application No. 2005/0148594. As reported therein, the formulations comprising amorphous fenofibric acid when administered to a subject, exhibit a bioavailability that is twice as high as a fenofibrate-containing capsule formulation described in Example 6 of said published application. Thereupon, in view of aforementioned described difference in solubility, the active ingredient, namely, fenofibrate, simply cannot be replaced with fenofibric acid in such dosage forms. WO 2004/054568 discloses a formulation comprising fenofibric acid or a physiologically acceptable salt thereof and optionally other active substances, a binder component comprising at least one enteric binder and optionally other physiologically acceptable excipients.

There is a need in the art for solid dosage forms containing fenofibric acid, salts of fenofibric acid and/or buffered fenofibric acid where the release of fenofibric acid, salts of fenofibric acid and/or buffers of fenofibric acid is controlled in such a way that when said solid dosage form is administered to a patient that the Cmax of said solid dosage form does not exceed 125% of the Cmax of a reference pharmaceutical composition containing fenofibrate. When the Cmax of said solid dosage forms does not exceed 125% of the Cmax of a reference pharmaceutical composition, then it would be expected that said solid dosage forms would provide a comparable safety profile to the reference pharmaceutical composition. For efficacy reasons, there is also a need in the art for solid dosage forms of fenofibric acid, salts of fenofibric acid and/or buffers of fenofibric acid that exhibit an AUC similar to the AUC of such reference pharmaceutical compositions.

Moreover, there is a need in the art for solid dosage forms of fenofibric acid, salts of fenofibric acid and/or buffered fenofibric acid that exhibit a lack of a significant food effect when administered to a patient under fed or fasted conditions. Such solid dosage forms would improve patient compliance by giving the patient the flexibility to take said solid dosage form under either fed or fasted conditions. Nonetheless, the time and resources needed to develop these solid dosage forms are significant. Solid dosage forms require testing in an appropriate animal model and/or in human subjects. In the event a solid dosage form fails to achieve an appropriate Cmax and/or AUC, a subsequent round of *in vitro* testing and *in vivo* testing may be required. Therefore, it would be useful to those skilled in the art if one or more models could be developed to describe the relationship and provide a correlation between an *in vitro* property of a solid dosage form and an *in vivo* response (such as, for example, food effect, bioequivalency and $C_{max}$). Such models would reduce the amount of time and resources required to develop such solid dosage forms. Moreover, such models may provide a formulator with a guide in developing and screening solid dosage forms.

<u>Summary of the Invention</u>

In one aspect, the present disclosure relates to a solid dosage form that comprises an active agent, wherein the active agent is at least one of 2-[4-(4-chlorobenzoyl)phenoxy]-2- methyl-propanoic acid, a salt of 2-[4-(4-chlorobenzoyl)phenoxy]-2-methyl-propanoic acid or a buffered 2-[4-(4-chlorobenzoyl)phenoxy]-2-methyl-propanoic acid, wherein a percentage of the dosage form dissolved in an *in vitro* dissolution at a single pH is: (a) less than or equal to 70% at thirty (30) minutes; (b) at least 0.9% at thirty (30) minutes and less than or equal to 70% at thirty (30) minutes; (c) less than or equal to 80% at sixty (60) minutes; (d) at least 7.0% at sixty (60) minutes and less than or equal to 80% at sixty (60) minutes; (e) at least 0.9% and less than or equal to 70% at thirty (30) minutes and is at least 7.0% and less than or equal to 80% at sixty (60) minutes; (f) less than or equal to 90% at ninety (90) minutes; or (g) at least 0.9% and less than or equal to 70% at thirty (30) minutes, at least 7.0% and less than or equal to 80% at sixty (60) minutes and less than or equal to 90% at ninety (90) minutes.

The above-described dosage form (namely, (a) - (g)), after administration to a human subject under fasting conditions, exhibits a $C_{max}$ that does not exceed 125% of a Cmax of a reference pharmaceutical composition. Specifically, the $C_{max}$ of the above-described dosage form (namely (a) - (o)) after administration to a human subject under fasting conditions is (1) less than the $C_{max}$ of a reference pharmaceutical composition; (2) at least 125% of the $C_{max}$ of the reference pharmaceutical composition; (3) at least 120% of the $C_{max}$ of the reference pharmaceutical composition; (4) at least 115% of the Cmax of the reference pharmaceutical composition; (5) at least 110% of the $C_{max}$ of the reference pharmaceutical composition; (6) at least 105% of the $C_{max}$ of the reference pharmaceutical composition; (7) at least 100% of the $C_{max}$ of the reference pharmaceutical composition; (8) at least 95% of the $C_{max}$ of the reference pharmaceutical composition; (9) at least 90% of the $C_{max}$ of the reference pharmaceutical composition; (10) at least 85% of the reference pharmaceutical composition; or (11) at least 80% of the $C_{max}$ of the reference pharmaceutical composition.

Moreover, the AUC above-described dosage form (namely, (a) - (g)) after administration to a human subject under fasting conditions is (1) at least 65% of an AUC of a reference pharmaceutical composition; (2) at least 70% of an AUC of a reference pharmaceutical composition; (3) at least 75% of an AUC of a reference pharmaceutical composition; (4) at least 80% of the AUC of the reference pharmaceutical composition; (5) at least 85% of the AUC of the reference pharmaceutical composition; (6) at least 90% of the AUC of the reference pharmaceutical composition; (7) at least 95% of the AUC of the reference pharmaceutical composition; (8) at least 100% of the AUC of the reference pharmaceutical composition; (9) at least 105% of the AUC of the reference pharmaceutical composition; (10) at least 110% of the AUC of the reference pharmaceutical composition; (11) at least 115% of the AUC of the reference pharmaceutical composition; (12) at least 120% of the AUC of the reference pharmaceutical composition; or (13) at least 125% of the AUC of the reference pharmaceutical composition.

In another aspect, the present disclosure relates to a solid dosage form that comprises an active agent, wherein the active agent is at least one of 2-[4-(4-chlorobenzoyl)phenoxy]-2- methyl-propanoic acid, a salt of 2-[4-(4-chlorobenzoyl)phenoxy]-2-methyl-propanoic acid or a buffered 2-[4-(4-chlorobenzoyl)phenoxy]-2-methyl-propanoic acid, wherein a percentage of the dosage form dissolved in an *in vitro* dissolution at single pH (a) at 0.5 hours is at least 15.0% and is less than or equal to 57.0%; (b) at one (1) hour is at least 40.0% and less than or equal to 70.0%; or (c) at 0.5 hours is at least 15.0% and is less than or equal to 57.0% and at one (1) hour is at least 40.0% and less than or equal to 70.0% and further wherein the dissolution profile of said solid dosage form follows a square root of time.

The above-described dosage form, after administration to a human subject under fasting conditions, is (1) at least 65% of an AUC of a reference pharmaceutical composition; (2) at least 70% of an AUC of a reference pharmaceutical composition; (3) at least 75% of an AUC of a reference pharmaceutical composition; (4) at least 80% of the AUC of the reference pharmaceutical composition; (5) at least 85% of the AUC of the reference pharmaceutical composition; (6) at least 90% of the AUC of the reference pharmaceutical composition; (7) at least 95% of the AUC of the reference pharmaceutical composition; (8) at least 100% of the AUC of the reference pharmaceutical composition; (9) at least 105% of the AUC of the reference pharmaceutical composition; (10) at least 110% of the AUC of the reference pharmaceutical composition; (11) at least 115% of the AUC of the reference pharmaceutical composition; (12) at least 120% of the AUC of the reference pharmaceutical composition; or (13) at least 125% of the AUC of the reference pharmaceutical composition.

Moreover, the AUC above-described dosage form after administration to a human subject under fasting conditions is (1) at least 65% of an AUC of a reference pharmaceutical composition; (2) at least 70% of an AUC of a reference pharmaceutical composition; (3) at least 75% of an AUC of a reference pharmaceutical composition; (4) at least 80% of the AUC of the reference pharmaceutical composition; (5) at least 85% of the AUC of the reference pharmaceutical composition; (6) at least 90% of the AUC of the reference pharmaceutical composition; (7) at least 95% of the AUC of the reference pharmaceutical composition; (8) at least 100% of the AUC of the reference pharmaceutical composition; (9) at least 105% of the AUC of the reference pharmaceutical composition; (10) at least 110% of the AUC of the reference pharmaceutical composition; (11) at least 115% of the AUC of the reference pharmaceutical composition; (12) at least 120% of the AUC of the reference pharmaceutical composition; or (13) at least 125% of the AUC of the reference pharmaceutical composition.

In another aspect, the present disclosure relates to a solid dosage form that comprises an active agent, wherein the active agent is at least one of 2-[4-(4-chlorobenzoyl)phenoxy]-2- methyl-propanoic acid, a salt of 2-[4-(4-chlorobenzoyl)phenoxy]-2-methyl-propanoic acid or a buffered 2-[4-(4-chlorobenzoyl)phenoxy]-2-methyl-propanoic acid, wherein a percentage of the dosage form dissolved in an in vitro dissolution at a single pH is: (a) at least 0.9% and less than or equal to 62% at 0.5 hours (thirty (30) minutes); (b) at least 7.0% and less than or equal to 71.0% at one (1) hour; or (c) at least 0.9% and less than or equal to 62% at 0.5 hours (thirty (30) minutes) and at least 7.0% and less than or equal to 71.0% at one (1) hour.

The above-described dosage form (namely, (a) - (c)), after administration to a human subject under fasting conditions, exhibits a $C_{max}$ that does not exceed 125% of a $C_{max}$ of a reference pharmaceutical composition. Specifically, the $C_{max}$ of the above-described dosage form (namely (a) - (o)) after administration to a human subject under fasting conditions is (1) less than the $C_{max}$ of a reference pharmaceutical composition; (2) at least 125% of the $C_{max}$ of the reference pharmaceutical composition; (3) at least 120% of the $C_{max}$ of the reference pharmaceutical composition; (4) at least 115% of the $C_{max}$ of the reference pharmaceutical composition; (5) at least 110% of the $C_{max}$ of the reference pharmaceutical composition; (6) at least 105% of the $C_{max}$ of the reference pharmaceutical composition; (7) at least 100% of the $C_{max}$ of the reference pharmaceutical composition; (8) at least 95% of the $C_{max}$ of the reference pharmaceutical composition; (9) at least 90% of the $C_{max}$ of the reference pharmaceutical composition; (10) at least 85% of the reference pharmaceutical composition; or (11) at least 80% of the $C_{max}$ of the reference pharmaceutical composition.

Moreover, the AUC above-described dosage form (namely, (a) - (c)) after administration to a human subject under fasting conditions is (1) at least 65% of an AUC of a reference pharmaceutical composition; (2) at least 70% of an AUC of a reference pharmaceutical composition; (3) at least 75% of an AUC of a reference pharmaceutical composition; (4) at least 80% of the AUC of the reference pharmaceutical composition; (5) at least 85% of the AUC of the reference pharmaceutical composition; (6) at least 90% of the AUC of the reference pharmaceutical composition; (7) at least 95% of the AUC of the reference pharmaceutical composition; (8) at least 100% of the AUC of the reference pharmaceutical composition; (9) at least 105% of the AUC of the reference pharmaceutical composition; (10) at least 110% of the AUC of the reference pharmaceutical composition; (11) at least 115% of the AUC of the reference pharmaceutical composition; (12) at least 120% of the AUC of the reference pharmaceutical composition; or (13) at least 125% of the AUC of the reference pharmaceutical composition.

In another aspect, the present disclosure relates to a solid dosage form that comprises an active agent, wherein the active agent is at least one of 2-[4-(4-chlorobenzoyl)phenoxy]-2- methyl-propanoic acid, a salt of 2-[4-(4-chlorobenzoyl)phenoxy]-2-methyl-propanoic acid or a buffered 2-[4-(4-chlorobenzoyl)phenoxy]-2-methyl-propanoic acid, wherein a percentage of the dosage form dissolved in an *in vitro* dissolution at a single pH at: (a) one (1) hour is at least 7.0%; (b) two (2) hours is at least 16.0%; (c) three (3) hours is at least 24.0%; (d) three and one-half (3.5) hours is at least 28.0%; (e) four (4) hours is at least 29.0%; (f) one (1) hour is at least 7.0% and at two (2) hours is at least 16.0%; (g) one (1) hour is at least 7.0%, at two (2) hours is at least 16.0% and at three (3) hours is at least 24.0%; (h) one (1) hour is at least 7.0%, at two (2) hours is at least 16.0%, at three (3) hours is at least 24.0% and at three and one-half (3.5) hours is at least 28.0%; (i) one (1) hour is at least 7.0%, at two (2) hours is at least 16.0%, at three (3) hours is at least 24.0%, at three and one-half (3.5) hours is at least 28.0% and at four (4) hours is at least 29.0%; (j) one (1) hour is less than or equal to 41.0%; (Ic) at two (2) hours is less than or equal to 79.0%; (1) one (1) hour is less than or equal to 41.0% and at two (2) hours is less than or equal to 79.0%; (m) one (1) hour is from 7.0% to 41.0%; (n) two (2) hours is from 16.0% to 79.0%; or (o) one (1) hour is from 7.0 % to 41.0% and at two (2) hours is from 16.0% to 79.0%.

The above-described dosage form (namely, (a) - (o)), after administration to a human subject under fasting conditions, exhibits a $C_{max}$ that does not exceed 125% of a $C_{max}$ of a reference pharmaceutical composition. Specifically, the $C_{max}$ of the above-described dosage form (namely (a) - (o)) after administration to a human subject under fasting conditions is (1) less than the $C_{max}$ of a reference pharmaceutical composition; (2) at least 125% of the $C_{max}$ of the reference pharmaceutical composition; (3) at least 120% of the $C_{max}$ of the reference pharmaceutical composition; (4) at least 115% of the $C_{max}$ of the reference pharmaceutical composition; (5) at least 110% of the $C_{max}$ of the reference pharmaceutical composition; (6) at least 105% of the $C_{max}$ of the reference pharmaceutical composition; (7) at least 100% of the $C_{max}$ of the reference pharmaceutical composition; (8) at least 95% of the $C_{max}$ of the reference pharmaceutical composition; (9) at least 90% of the $C_{max}$ of the reference pharmaceutical composition; (10) at least 85% of the reference pharmaceutical composition; or (11) at least 80% of the $C_{max}$ of the reference pharmaceutical composition.

Moreover, the AUC above-described dosage form (namely, (a) - (o)) after administration to a human subject under fasting conditions is (1) at least 65% of an AUC of a reference pharmaceutical composition; (2) at least 70% of an AUC of a reference pharmaceutical composition; (3) at least 75% of an AUC of a reference pharmaceutical composition; (4) at least 80% of the AUC of the reference pharmaceutical composition; (5) at least 85% of the AUC of the reference pharmaceutical composition; (6) at least 90% of the

AUC of the reference pharmaceutical composition; (7) at least 95% of the AUC of the reference pharmaceutical composition; (8) at least 100% of the AUC of the reference pharmaceutical composition; (9) at least 105% of the AUC of the reference pharmaceutical composition; (10) at least 110% of the AUC of the reference pharmaceutical composition; (11) at least 115% of the AUC of the reference pharmaceutical composition; (12) at least 120% of the AUC of the reference pharmaceutical composition; or (13) at least 125% of the AUC of the reference pharmaceutical composition.

In still another aspect, the present disclosure relates to a solid dosage form that comprises an active agent, wherein the active agent is at least one of 2-[4-(4-chlorobenzoyl)phenoxy]-2-methyl-propanoic acid, a salt of 2-[4-(4-chlorobenzoyl)phenoxy]- 2-methyl-propanoic acid or a buffered 2-[4-(4-chlorobenzoyl)phenoxy]-2-methyl-propanoic acid, wherein a percentage of the dosage form dissolved in an *in vitro* dissolution at a single pH at: (a) one (1) hour is at least 9.0%; (b) two (2) hours is at least 21.0%; (c) three (3) hours is at least 34.0%; (d) three and one-half (3.5) hours is at least 39.0%; (e) four (4) hours is at least 44.0%; (f) one (1) hour is at least 7.0% and at two (2) hours is at least 21.0%; (g) one (1) hour is at least 9.0%, at two (2) hours is at least 21.0% and at three (3) hours is at least 34.0%; (h) one (1) hour is at least 9.0%, at two (2) hours is at least 21.0%, at three (3) hours is at least 34.0% and at three and one-half (3.5) hours is at least 39.0%; (i) one (1) hour is at least 9.0%, at two (2) hours is at least 21.0%, at three (3) hours is at least 34.0%, at three and one-half (3.5) hours is at least 39.0% and at four (4) hours is at least 44.0%; (j) one (1) hour is from 9.0% to 41.0%; (k) two (2) hours is from 21.0% to 79.0%; or (1) one (1) hour is from 9.0 % to 41.0% and at two (2) hours is from 21.0% to 79.0%.

The above-described dosage form (namely, (a) - (1)), after administration to a human subject under fasting conditions, exhibits a $C_{max}$ that does not exceed 125% of a $C_{max}$ of a reference pharmaceutical composition. Specifically, the $C_{max}$ of the above-described dosage form (namely (a) - (o)) after administration to a human subject under fasting conditions is (1) less than the $C_{max}$ of a reference pharmaceutical composition; (2) at least 125% of the $C_{max}$ of the reference pharmaceutical composition; (3) at least 120% of the $C_{max}$ of the reference pharmaceutical composition; (4) at least 115% of the $C_{max}$ of the reference pharmaceutical composition; (5) at least 110% of the $C_{max}$ of the reference pharmaceutical composition; (6) at least 105% of the $C_{max}$ of the reference pharmaceutical composition; (7) at least 100% of the $C_{max}$ of the reference pharmaceutical composition; (8) at least 95% of the $C_{max}$ of the reference pharmaceutical composition; (9) at least 90% of the $C_{max}$ of the reference pharmaceutical composition; (10) at least 85% of the reference pharmaceutical composition; or (11) at least 80% of the $C_{max}$ of the reference pharmaceutical composition.

Moreover, the AUC above-described dosage form (namely, (a) - (1)) after administration to a human subject under fasting conditions is (1) at least 65% of an AUC of a reference pharmaceutical composition; (2) at least 70% of an AUC of a reference pharmaceutical composition; (3) at least 75% of an AUC of a reference pharmaceutical composition; (4) at least 80% of the AUC of the reference pharmaceutical composition; (5) at least 85% of the AUC of the reference pharmaceutical composition; (6) at least 90% of the AUC of the reference pharmaceutical composition; (7) at least 95% of the AUC of the reference pharmaceutical composition; (8) at least 100% of the AUC of the reference pharmaceutical composition; (9) at least 105% of the AUC of the reference pharmaceutical composition; (10) at least 110% of the AUC of the reference pharmaceutical composition; (11) at least 115% of the AUC of the reference pharmaceutical composition; (12) at least 120% of the AUC of the reference pharmaceutical composition; or (13) at least 125% of the AUC of the reference pharmaceutical composition.

In still yet another aspect, the present disclosure relates to a solid dosage form that comprises an active agent, wherein the active agent is at least one of 2-[4-(4-chlorobenzoyl)phenoxy]-2-methyl-propanoic acid, a salt of 2-[4-(4-chlorobenzoyl)phenoxy]- 2-methyl-propanoic acid or a buffered 2-[4-(4-chlorobenzoyl)phenoxy]-2-methyl-propanoic acid, wherein said solid dosage exhibits a difference between an *in vitro* dissolution at a dual pH and an *in vitro* dissolution at a single pH of (a) -10.0 to 17.0; (b) -10.0 to 9.0; or (c)-10.0 to 2.0; and further wherein said dissolution at the dual pH and at the single pH are each determined at two (2) hours. The AUC of the above-described solid dosage form, upon oral administration to a human subject in a fed state, does not differ substantially, when compared to an AUC of said dosage form upon oral administration to a human subject in a fasting state. Moreover, the AUC of said dosage form under fed conditions divided by the AUC of said dosage form under fasting conditions is between (a) 0.7 and 1.43; or (b) 0.80 and 1.25.

Additionally, the above-described dosage form, after administration to a human subject under fasting conditions, exhibits a $C_{max}$ that does not exceed 125% of a $C_{max}$ of a reference pharmaceutical composition. Specifically, the $C_{max}$ of the above-described dosage form after administration to a human subject under fasting conditions is (1) less than the $C_{max}$ of a reference pharmaceutical composition; (2) at least 125% of the $C_{max}$ of the reference pharmaceutical composition; (3) at least 120% of the $C_{max}$ of the reference pharmaceutical composition; (4) at least 115% of the $C_{max}$ of the reference pharmaceutical composition; (5) at least 110% of the $C_{max}$ of the reference pharmaceutical composition; (6) at least 105% of the $C_{max}$ of the reference pharmaceutical composition; (7) at least 100% of the $C_{max}$ of the reference pharmaceutical composition; (8) at least 95% of the $C_{max}$ of the reference pharmaceutical composition; (9) at least 90% of the $C_{max}$ of the reference pharmaceutical composition; (10) at least 85% of the reference pharmaceutical composition; or (11) at least 80% of the $C_{max}$ of the reference pharmaceutical composition.

In still yet another aspect, the present disclosure relates to a solid dosage form that comprises an active agent, wherein the active agent is at least one of 2-[4-(4-chlorobenzoyl)phenoxy]-2-methyl-propanoic acid, a salt of 2-[4-(4-chlorobenzoyl)phenoxy]- 2-methyl-propanoic acid or a buffered 2-[4-(4-chlorobenzoyl)phenoxy]-2-methyl-propanoic acid, wherein when said solid dosage has a Predicted ΔRelative $C_{max}$ of from (a) -0.2 to +0.8; or (b) -0.2 to +0.2. The AUC of the above-described solid dosage form, upon oral administration to a human subject in a fed state, does not differ substantially, when compared to an AUC of said dosage form upon oral administration to a human subject in a fasting state. Moreover, the AUC of said dosage form under fed conditions divided by the AUC of said dosage form under fasting conditions is between (a) 0.7 and 1.43; or (b) 0.80 and 1.25. Additionally, the above-described dosage form, after administration to a human subject under fasting conditions, exhibits a $C_{max}$ that does not exceed 125% of a $C_{max}$ of a reference pharmaceutical composition. Specifically, the $C_{max}$ of the above-described dosage form after administration to a human subject under fasting conditions is (1) less than the $C_{max}$ of a reference pharmaceutical composition; (2) at least 125% of the $C_{max}$ of the reference pharmaceutical composition; (3) at least 120% of the $C_{max}$ of the reference pharmaceutical composition; (4) at least 115% of the $C_{max}$ of the reference pharmaceutical composition; (5) at least 110% of the $C_{max}$ of the reference pharmaceutical composition; (6) at least 105% of the

$C_{max}$ of the reference pharmaceutical composition; (7) at least 100% of the $C_{max}$ of the reference pharmaceutical composition; (8) at least 95% of the $C_{max}$ of the reference pharmaceutical composition; (9) at least 90% of the $C_{max}$ of the reference pharmaceutical composition; (10) at least 85% of the reference pharmaceutical composition; or (11) at least 80% of the $C_{max}$ of the reference pharmaceutical composition.

The present invention relates to novel oral pharmaceutical formulations that comprise at least one active agent, wherein the active agent is a salt of 2-[4-(4- chlorobenzoyl)phenoxy]-2-methyl-propanoic acid with choline.

The oral pharmaceutical formulations of the invention further comprise at least one enteric coating.

In a preferred aspect, the present invention relates to **oral** pharmaceutical formulations as defined above which are novel modified release oral pharmaceutical formulations . Optionally, said formulations can comprise at least one rate-controlling mechanism..

In a further aspect, the modified release oral pharmaceutical compositions of the present invention can comprise at least one core. The core of the formulation of the present invention can contain at least one active agent. For example, the core can comprise at least a choline salt of 2-[4-(4-chlorobenzoyl)phenoxy]-2-methyl-propanoic acid . Optionally, the core can also comprise at least one rate-controlling mechanism.

The above-described core can be surrounded by or coated with at least one non-rate controlling layer, at least one rate-controlling mechanism, at least one enteric coating or any combinations thereof. For example, the core can be surrounded by an enteric coating. Alternatively, the core can be surrounded or coated with a non-rate-controlling layer. Optionally, this non-rate controlling layer can be surrounded or coated with a rate-controlling mechanism, an enteric coating or a combination of a rate-controlling mechanism and an enteric coating. Alternatively, the core can be surrounded or coated with a rate-controlling mechanism. This rate-controlling mechanism can be surrounded or coated with a non-rate- controlling layer, an enteric coating or a combination of a non-rate-controlling mechanism and an enteric coating.

In another aspect, the core can comprise an inert substrate. This inert substrate can be coated with a choline salt of 2-[4-(4-chlorobenzoyl)phenoxy]-2-methyl-propanoic acid . This coated substrate can be surrounded or coated with at least one non-rate controlling layer, at least one rate-controlling mechanism, at least one enteric coating or any combinations thereof. For example, the core can be surrounded by an enteric coating. Alternatively, the substrate can be surrounded or coated with a non-rate-controlling layer. Optionally, this non-rate controlling layer can be surrounded or coated with a rate-controlling mechanism, an enteric coating or a combination of a rate-controlling mechanism and an enteric coating. Alternatively, the substrate can be surrounded or coated with a rate- controlling mechanism. This rate-controlling mechanism can be surrounded or coated with a non-rate-controlling layer, an enteric coating or a combination of a non-rate-controlling mechanism and an enteric coating.

The modified release formulations described herein can contain at least one pharmaceutically acceptable excipient. Any pharmaceutically acceptable excipient that is appropriate for use in the formulation of the present invention can be used or included, such as, but not limited to, fillers, binders, lubricants, glidants, solubility enhancing agents, suspending agents, sweetness and/or flavoring agents, preservatives, buffers, wetting agents, distintegrating agents, effervescent agents, surfactants, humectants, solution retarders and combinations thereof.

The at least one rate-controlling mechanism described herein can be used in a mixture containing active agents or a coating (membrane) surrounding the active agents. When used as a mixture containing the active agents, the rate-controlling mechanism used in the formulation of the present invention can be hydrophilic agents, hydrophobic agents or combinations thereof. Additionally, the rate-controlling mechanism of the present invention may optionally include any pharmaceutically acceptable excipient that can help modulate the hydrophilicity and/or hydrophobicity of the hydrophilic and/or hydrophobic agents. Examples of hydrophilic agents that can be used include, but are not limited to, celluloses (such as hydroxypropyl methylcelluloses, hydroxypropyl cellulose and hydroxyethyl celluloses), polyethylene oxides, polyethylene glycols, xanthan gums, alginates, polyvinyl pyrrolidones, starches, cross-linked homopolymers and copolymers of acrylic acid and combinations thereof. Hydrophobic agents that can be used include, but are not limited to, waxes or water-insoluble agents. Examples of waxes that can be used include, but are not limited to, natural and synthetic waxes, such as, carnauba wax, bees wax, candelilla wax, paraffin waxes and combinations thereof. Water insoluble agents that can be used include, but are not limited to, ammoniomethacrylate copolymers (such as Eudragit® RL100 and RS100), cellulose, ethylcellulose, cellulose acetates, cellulose acetate butyrate, cellulose acetate propionate, methacrylic ester copolymers (such as Eudragit® NE30D), microcrystalline cellulose and dibasic calcium phosphate and combinations thereof. When used as a coating (membrane) surrounding the active agent(s), the rate-controlling mechanism includes, but is not limited to, ethylcellulose (such as Surelease® and Aquacoat® ECD), ammoniomethacrylate copolymers (such as Eudragit® RL30D and RS30D) and methacrylic ester copolymers (such as Eudragit® NE30D).

As described previously herein, the formulation of the present invention can contain one or more non-rate-controlling layers, membranes or coatings. The location of the non-rate-controlling layer in the formulation is not critical. For example, the non-rate-controlling layer may be present between the at least one core and an enteric coating or a rate-controlling mechanism. Alternatively, the non-rate-controlling layer may surround or coat an enteric coating or a rate-controlling mechanism. The non-rate-controlling layer can be made of one or more polymers, as well as, other ingredients known in the art, such as, but not limited to, plasticizers, pigments/opacifiers, etc. Examples of polymers that can be used include, but are not limited to, hydroxypropyl methylcellulose, hydroxypropyl cellulose, methylcellulose, ethylcellulose, polyvinyl alcohol, and polyethylene glycol. Examples of plasticizers that can be used include, but limited to, polyethylene glycol(s), glycerin, triacetin, triethyl citrate, diethyl phthalate and mineral oils. Examples of pigments/opacifiers that can be used include, but are not limited to, water soluble dyes, pigments, and natural products.

As also discussed previously herein, the formulations of the present invention also include at least one enteric coating. Any enteric coating can be used in the present invention, including, but not limited to, solutions or dispersions of methacrylic acid and methacrylic ester copolymers, cellulose acetate phthalate, hydroxypropyl methylcellulose phthalate, hydroxypropyl methylcellulose acetate succinate, polyvinyl acetate phthalate, ethyl acrylate/methacrylic acid copolymers, cellulose acetate trimellitate, shellac and combinations thereof. Additionally, the enteric coating used in the formulations of the present invention can be formed as a single or multiple layers. The thickness of the coating can be readily determined by those skilled in the art, but must be sufficient to protect the formulation in the acidic environment of the stomach.

The formulations of the present invention can further contain active agents other than a choline salt of 2-[4-(4-chlorobenzoyl)phenoxy]-2-methyl-propanoic acid. The location of these other agents within the formulation is not critical. Alternatively, the formulations of the present invention can be co-administered with one or more separate dosage forms that contain one or more agents other than a choline salt of 2-[4-(4- chlorobenzoyl)phenoxy]-2-methyl-propanoic acid. Examples of other agents that can be used, include, but are not limited to, lipid regulating agents (such as, but not limited to, atorvastatin, simvastatin, fluvastatin, pravastatin, lovastatin, cerivastatin, rosuvastatin, pitavastatin, clofibric acid, niacin/nicotinic acid, torcetrapib, colestipol, omega-3 acid ethyl esters, colesevelam, cholestyramine, ezetimibe, MD-0727, gemfibrozil or probucol); anti-hypertensive agents (such as, but not limited to, amlodipine, benazepril, benidipine, candesartan, captopril, carvedilol, darodipine, dilitazem, diazoxide, doxazosin, enalapril, epleronone, eprosartan, felodipine, fenoldopam, fosinopril, guanabenz, iloprost, irbesartan, isradipine, lercardinipine, lisinopril, losartan, minoxidil, nebivolol, nicardipine, nifedipine, nimodipine, nisoldipine, omapatrilat, phenoxybenzamine, prazosin, quinapril, reserpine, semotiadil, sitaxsentan, terazosin, telmisartan, labetolol, valsartan, triamterene, metoprolol, methyldopa, ramipril, olmesartan, timolol, verapamil, clonidine, nadolol, bendromethiazide, torsemide, hydrochlorothiazide, spinronolactone, perindopril, hydralazine, betaxolol, furosimide, penbutolol, acebutolol, atenolol, bisoproloL nadolol, penbutolol, pindolol, propranolol, timolol, indapamide, trandolopril, amiloride, moexipril, metolozone, or valsartan); antidiabetic agents (such as, but not limited to, acarbose, oral insulin, acetohexamide, chlorpropamide, ciglitazone, farglitazar, glibenclamide, gliclazide, glipizide, glucagon, glyburide, glymepiride, miglitol, pioglitazone, nateglinide, pimagedine, repaglinide, rosiglitazone, tolazamide, tolbutamide, triampterine or troglitazone); weight-loss agents (such as, but not limited to, phentermine, phendimetrazine, benzphetamine, diethylpropion, sibutramine, orlistat or rimonabant); antiretro viral agents (such as but not limited to, amprenavir, tiprinavir, lamivudine, indinavir, emtricitabine, abacavir, enfuvirtide, saquinavir, lopinavir, ritonavir, fosamprenavir, delaviradine mesylate, zidovudine, atazanavir, efavirenz, tenofivir, emtricitabine, didanosine, nelfmavir, nevirapine, or stavudine); anti-platelet agents (such as, but not limited to, aspirin, cilostazol, or pentoxifylline); or vitamins, minerals or combinations of vitamins and minerals (such as, but not limited to folic acid, calcium, or iron).

Brief Description of the Figures

Figure 1 shows the prediction of *in vivo* $C_{max}$ food effect from *in vitro* dissolution differences at 0.5, 1.0 and 2.0 hour time points.

Figure 2 shows the prediction of *in vivo* $C_{max}$ food effect from *in vitro* dissolution differences 2.0 hours.

Figure 3 shows the dissolution profiles of Formulations 1-13 using a single pH method.

Figure 4 shows the dissolution profiles of Formulations 1-13 using a dual pH method.

Detailed Description of the Invention

I. Definitions

As used in this specification and the appended claims, the singular forms "a," "an" and "the" include plural references unless the context clearly dictates otherwise. Thus, for example, reference to "an active agent" includes a single active agent as well two or more different active agents in combination, reference to "an excipient" includes mixtures of two or more excipients as well as a single excipient, and the like.

In describing and claiming the present invention, the following terminology will be used in accordance with the definitions set out below.

As used herein, the term "about" is used synonymously with the term "approximately." Illustratively, the use of the term "about" indicates that values slightly outside the cited values, namely, plus or minus 10%. Such dosages are thus encompassed by the scope of the claims reciting the terms "about" and "approximately."

As used herein, the term "AUC" refers to the area under the plasma concentration time curve of the active agent and which is calculated using the trapezoidal rule. The term "$AUC_t$" means the area under the plasma concentration time curve from time 0 to 120 hours after administration in units of ng•h/mL as determined using the trapezoidal rule. The term "$AUC_\infty$" means the area under the plasma concentration time curve from time 0 to infinite time. $AUC_\infty$ is calculated as $AUC_t + LMT/(-\beta)$, where "LMT" is the last measurable plasma concentration and $\beta$ is the terminal phase elimination rate constant. Unless otherwise noted herein, the reported value for the AUC is the central value of the AUC.

As used herein, the terms "active agent," "pharmacologically active agent," and "drug" are used interchangeably herein to refer to 2-[4-(4-chlorobenzoyl)phenoxy]-2-methyl-propanoic acid. The terms also encompass salts and buffered 2-[4-(4-chlorobenzoyl)phenoxy]-2-methyl-propanoic acid. When the terms "active agent," "pharmacologically active agent" and "drug" are used, it is to be understood that Applicants intend to include 2-[4-(4-chlorobenzoyl)phenoxy]-2-methyl-propanoic acid *per se* as well as salts and buffered 2-[4-(4-chlorobenzoyl)phenoxy]-2-methyl-propanoic acid. Salts of 2-[4-(4-chlorobenzoyl)phenoxy]-2-methyl-propanoic acid include, but are not limited to, choline, ethanolamine, diethanolamine, dicyclohexylamine, tromethamine, lysine, piperazine, calcium and tromethamine. Examples of counterions that can be used to provide buffered 2-[4-(4-chlorobenzoyl)phenoxy]-2-methyl-propanoic acid, include, but are not limited to, calcium hydroxide, choline hydroxide, diethylethanolamine, diethanolamine, ethylenediamine, guanidine, magnesium hydroxide, meglumine, ethanolamine, piperazine, peperidine, sodium hydroxide, triethylamine, tromethamine, benzathine, benzene-ethanamine, adenine, aluminum hydroxide, ammonium hydroxide, cytosine, diethylamine, glucosamine, guanine, nicotinamide, potassium hydroxide, zinc hydroxide, hydrabamine, tributylamine, deanol, epolamine, lithium hydroxide, procaine, pyridoxine, triethanolamine, ornithine, glycine, lysine, arginine, valine, serine, proline, aspartic acid, alanine, isoleucine, leucine, methionine or threnine. The solid state form of the active agent used in preparing the solid dosage forms of the present invention is not critical. For example, active agent used in preparing the solid dosage form can be amorphous or crystalline. The final dosage form contains at least a detectable amount of crystalline active agent. The crystalline nature of the active agent can be detected using powder X-ray diffraction analysis, by differential scanning calorimetry or any other techniques known in the art.

As described herein, two products, solid dosage forms or methods are considered to be "bioequivalent" if the 90% Confidence Interval ("CI") for comparing the AUCs between two formulations is between 0.70 and 1.43, more preferably, the CI is between 0.80 and 1.25.

As used herein, the term "CL/F" refers to apparent oral clearance and is calculated by dividing the dose, by the AUC.

As used herein, the term "$C_{max}$" refers to the maximum observed plasma concentration of 2-[4-(4-chlorobenzoyl)phenoxy]-2-methyl-propanoic acid. Unless otherwise noted herein, the reported value for the $C_{max}$ is the central value of the $C_{max}$.

As used herein, the term "delayed release" refer to a type of modified release wherein a drug dosage form exhibits a time delay between oral administration of the drug dosage form and the release of the drug from said dosage form. Pulsed release systems (also known as pulsatile drug release") and the use of enteric coatings, which are well known to those skilled in the art, are examples of delayed release mechanisms.

As used herein, the phrase "delta dissolution time" or "$\Delta diss_t$" refers to the following formula:

$$\Delta diss_t = \text{dissolution at time t (using the dual pH method)} - \text{dissolution at time t (using the single pH method).}$$

For example, time ("t") can be 0.5 hours (30 minutes), 1.0 hour, 2.0 hours, etc.

As used herein, "$\Delta diss_{0.5hr}$" refers to the value or number obtained from the difference in release as measured using an *in vitro* dissolution test at a dual pH at 0.5 hours (30 minutes) for a dosage form and an *in vitro* dissolution at a single pH at 0.5 hours (30 minutes) for said same dosage form.

As used herein, "$\Delta diss_{1.0hr}$" refers to the value or number obtained from the difference in release as measured using an *in vitro* dissolution test at a dual pH at one (1) hour for a dosage form and an *in vitro* dissolution at a single pH at one (1) hour for said same dosage form.

As used herein, "$\Delta diss_{2.0hr}$" refers to the value or number obtained from the difference in release as measured using an *in vitro* dissolution test at a dual pH at two (2) hours for a dosage form and an *in vitro* dissolution at a single pH at two (2) hours for said same dosage form.

As used herein, the phrase "dissolution at a dual pH", "a dual pH" or a "dual pH system" as used interchangeably herein, refers to the method described in Table 1 below:

Table 1

| Parameter | Condition |
|---|---|
| Apparatus | USP Apparatus 2 (USP 29 NF 24) |
| Agitation | 50 RPM±4% |
| Medium | Two Stages:<br>Acid Stage:<br>• 500 mL of 0.05 M sodium phosphate buffer + 0.2 M NaCl, 500 mL, pH 3.5 ± 0.05 maintained at 37 ± 0.5°C for 2 hours<br>Buffer Stage<br>• Followed by 400 mL of 0.05 M sodium phosphate (pH approximately 11.5) added to the Acid Stage media for a total volume of 900 mL and a final pH 6.8 maintained at 37 ± 0.5°C |
| Sampling Time Points | 2.25 - 12 hours (Sampling times start from dropping of the dosage forms in 0.05 M sodium phosphate buffer + 0.2 M NaCl (pH 3.5)) |
| UV Spectrophotometry Analysis | At 300 nm |

For an avoidance of a doubt, in a dual pH system, the measurement of the percentage (%) of an active agent dissolved in said system begins after a dosage form or composition has been exposed for two (2) hours in the acid stage medium, pH 3.5. For example, the amount of active agent X dissolved at 30 minutes in a dual pH system is measured two (2) hours and thirty (30) minutes after the acid stage medium was added. The dosage form or composition containing active agent X spends an initial two (2) hours in the acid stage. After the dosage form has been exposed to two (2) hours in the acid stage, pH 3.5, 400 mL of 0.05 M sodium phosphate (pH approximately 11.5) is added to the 500 mL of 0.05 M sodium phosphate buffer +0.2 M NaCl (pH 3.5) yielding 0.05 M sodium phosphate buffer, pH 6.8 ±0.05. The amount of active agent dissolved (namely the percentage (%) dissolution) is measured thirty (30) minutes after the buffer stage medium was added. The percentage (%) dissolution measured under this dual pH method was normalized to the strength of the dosage form.

As used herein, the phrase "dissolution at a single pH", "a single pH" or a "single pH system", as used interchangeably herein, refers to the method described in Table 2 below:

Table 2

| Parameter | Condition |
|---|---|
| Apparatus | USP Apparatus 2 (USP 29 NF 24) |
| Agitation | 50 RPM±4% |
| Medium | 0.05 M sodium phosphate buffer 900 mL, pH 6.8 ± 0.05 maintained at 37 ± 0.5°C |
| Sampling Time Points | 30 minutes to 10 hours |
| UV Spectrophotometry Analysis | At 300 nm |

The percentage (%) dissolution measured under this dual pH method was normalized to the strength of the dosage form.

By an "effective amount" or a "therapeutically effective amount" of an active agent is meant a nontoxic but sufficient amount of the active agent to provide the desired effect. The amount of active agent that is "effective" will vary from subject to subject, depending on the age and general condition of the individual, the particular active agent or agents, and the like. Thus, it is not always possible to specify an exact "effective amount." However, an appropriate "effective amount" in any individual case may be determined by one of ordinary skill in the art using routine experimentation.

As used herein, the term "extended release" or "sustained release" refers to a drug formulation that provides for gradual release of a drug over an extended period of time.

As used herein, a "fasted" patient, "fasting conditions" or "fasting" refers to a patient who has not eaten any food, i.e., who has fasted for at least 10 hours before the administration of the oral formulation of the present invention comprising at least one active agent and who does not eat any food and continues to fast for at least 4 hours after the administration of the formulation. The formulation is preferably administered with 240 ml of water during the fasting period, and water can be allowed *ad libitum* up to 1 hour before and 1 hour after ingestion.

As used herein, a "fed patient", "fed conditions" or "fed" refers to a patient who has fasted for at least 10 hours overnight and then has consumed an entire test meal beginning 30 minutes before the first ingestion of the test formulations. The formulation of the present invention is administered with 240 ml of water within 5 minutes after completion of the meal. No food is then allowed for at least 4 hours post-dose. Water can be allowed *ad libitum* up to 1 hour before and 1 hour after ingestion. A high fat test meal provides approximately 1000 calories to the patient of which approximately 50% of the caloric content is derived from fat content of the meal. A representative high fat high calorie test meal comprises 2 eggs fried in butter, 2 strips of bacon, 2 slices of toast with butter, 4 ounces of hash brown potatoes and 8 ounces of whole milk to provide 150 protein calories, 250 carbohydrate calories and 500 to 600 fat calories. High fat meals can be used in clinical effect of food studies of 2-[4-(4-chlorobenzoyl)phenoxy]-2-methyl-propanoic acid. A patient who receives such a high fat test meal is referred to herein as being under "high fat fed conditions". A low fat test meal provides approximately 500 calories to the patient of which approximately 30% of the caloric content is derived from fat content of the meal. A patient who receives such a low fat test meal is referred to herein as being under "low fat fed conditions".

As used herein, the terms "formulation", "form" or "dosage form" as used interchangeably herein, denotes any form of a pharmaceutical composition that contains an amount of active agent sufficient to achieve the desired therapeutic effect. The frequency of administration that will provide the most effective results in an efficient manner without overdosing will vary with the characteristics of the particular active agent, including both its pharmacological characteristics and its physical characteristics.

As used herein, the term "inert substrate" refers to (a) water insoluble substrates or seeds comprising different oxides, celluloses, organic polymers and other materials, alone or in mixtures; or (b) water soluble substrates or seeds comprising different inorganic salts, sugars, non-pareils and other materials, alone or in mixtures.

As used herein, the term "membrane" refers to a film or layer that is permeable to aqueous solutions or bodily fluids and may also be permeable to the active agent.

As used herein, the term "modified" refers to a drug containing formulation in which release of the drug is not immediate (See, for example, Guidance for Industry SUPAC-MR: Modified Release Solid Oral Dosage Forms, Scale-Up and Postapproval Changes: Chemistry, Manufacturing, and Controls; In Vitro Dissolution, Testing and In Vivo Bioequivalence Documentation, U.S. Department of Health and Human services, Food and Drug Administration, Center for Drug Evaluation and Research ("CDER"), September 1997 CMC 8, page 34, herein incorporated by reference.). In a modified formulation, administration of said formulation does not result in immediate release of the drug or active agent into an absorption pool. The term is used interchangeably with "nonimmediate release" as defined in Remington: The Science and Practice of Pharmacy, Nineteenth Ed. (Easton, Pa.: Mack Publishing Company, 1995). As used herein, the term "modified release" includes extended release, sustained release, delayed release, and controlled release formulations.

As used herein, the phrase "Observed $\Delta_{Relative}\, C_{max}$" refers to the following formula:

$$\text{Observed } \Delta_{\text{Relative}}\, C_{\text{max}} = \text{Relative } C_{\text{max}} \text{ Under Fed Conditions - Relative } C_{\text{max}}$$

$$\text{Under Fasting Conditions}$$

As used herein, the phrase "pharmaceutically acceptable," such as in the recitation of a "pharmaceutically acceptable excipient," or a "pharmaceutically acceptable additive," is meant a material that is non-toxic or otherwise physiologically acceptable.

As used herein, the term "reference pharmaceutical composition" or "Reference" refers to a capsule containing 200 mg of 2-[4-(4-chlorobenzoyl)phenoxy]-2-methyl-propanoic acid,1-methylethyl ester. The reference pharmaceutical composition is orally administered to a human subject under low fat fed conditions. The reference pharmaceutical composition is TRICOR®, Abbott Laboratories, Abbott Park, Illinois.

As used herein, the term "Relative $C_{max}$" refers to the following formula:

$$\text{Relative C max} = \frac{\text{Observed C max}}{\text{Cmax Observed in a Reference Pharmaceutical Composition}}$$

The "Observed $C_{max}$" in the above formula is the $C_{max}$ of the test dosage form (such as, but not limited to, a dosage form of the present invention after oral administration under fed or fasting conditions).

As used herein, the term "similarity factor" or "$f_2$" refers to the following formula:

$$f_2 = 50 \text{ LOG } \{[1+1/n\ \Sigma_{t=1}^{n}(R_t - T_t)^2]^{-0.5} \text{ x } 100\}$$

where LOG=logarithm to base 10, n=the number of sampling time points, $\Sigma$ = summation over time points, $R_t$= mean dissolution at time point t of the reference pharmaceutical formulation and $T_t$= mean dissolution at time point t of the test dosage form (such as, but not limited to, a solid dosage form of the present invention).

An $f_2$ value between 50 and 100 suggests that two dissolution profiles are similar. A detailed discussion of the similarity factor or $f_2$ can be found in the Guidance for Industry SUPAC-MR: Modified Release Solid Oral Dosage Forms, Scale-Up and Postapproval Changes: Chemistry, Manufacturing, and Controls; In Vitro Dissolution, Testing and In Vivo Bioequivalence Documentation, U.S. Department of Health and Human services, Food and Drug Administration, Center for Drug Evaluation and Research ("CDER"), September 1997 CMC 8, pages 32-33.

As used herein, the term "subject" refers to an animal, preferably a mammal, including a human or non-human. The terms patient and subject may be used interchangeably herein.

As used herein, the term "$T_{max}$" refers to the time to the maximum observed plasma concentration.

As used herein, the terms "treating" and "treatment" refer to reduction in severity and/or frequency of symptoms, elimination of symptoms and/or underlying cause, prevention of the occurrence of symptoms and/or their underlying cause, and improvement or remediation of damage. Thus, for example, "treating" a patient involves prevention of a particular disorder or adverse physiological event in a susceptible individual as well as treatment of a clinically symptomatic individual by inhibiting or causing regression of a disorder or disease.

The present invention relates to solid dosage forms comprising at least one active agent. The solid dosage forms of the present invention achieve a number of objects. First, the solid dosage forms of the present invention, when administered to a human subject under fasting conditions, achieve a $C_{max}$ that does not exceed the $C_{max}$ of a reference pharmaceutical composition. Such solid dosage forms provide a comparable safety profile to the reference pharmaceutical composition. Second, some of the solid dosage forms of the present invention when administered to a human subject exhibit an AUC that does not differ substantially from the AUC of the reference pharmaceutical composition. Thus, such solid dosage forms exhibit an efficacy that is similar to that of the reference pharmaceutical composition. Third, some of the solid dosage forms of the present invention do not exhibit a significant food effect when administered to a subject under fed and fasting conditions. Such solid dosage forms lead to increased subject convenience which leads to increasing subject compliance since the subject does not need to ensure that they are taking the dosage form either with or without food. This is significant, because when there is poor subject compliance, the medical condition for which the drug is being prescribed may not be properly managed.

The present <u>disclosure</u> also provides a model for determining whether a newly developed solid dosage form is likely to be bioequivalent to a reference pharmaceutical composition based on a correlation between *in vitro* dissolution and bioequivalency. Additionally, the present disclosure further provides a model for determining whether a newly developed solid dosage form is likely to exhibit a significant food effect when administered to a human subject. Moreover, the present disclosure also provides a model for predicting the $C_{max}$ of a newly developed solid dosage form based on a correlation between in *vitro* dissolution and $C_{max}$. The models described herein reduce the time and resources needed to develop such solid forms.

II. <u>The Dosage Forms of the Present Invention</u>

A. <u>Dissolution</u>

The dissolution values shown herein were determined with a conventional dosage form containing either 130 mg or 135 mg of active agent, unless otherwise noted.

In one aspect, the present disclosure relates to dosage forms comprising at least one active agent, where the percentage (%) of the active agent of the dosage form dissolved in an *in vitro* dissolution at a single pH is as shown below in Tables 3 and 4. The dissolution values shown in Table 3 were determined using the single pH method with a conventional capsule containing 135 mg of 2-[4-(4-chlorobenzoyl)phenoxy]-2-methyl-propanoic acid (hereinafter "Neat Drug"). Based on the $C_{max}$ values in Tables 25-30 in the Examples, the $C_{max}$ of the Neat Drug is expected to exceed the $C_{max}$ of the Reference Pharmaceutical Composition. The upper limit of the dissolution values shown in Table 4 is from Table 3. The lower limits of the dissolution values shown in Table 4 were determined based on the similarity factor calculations using Formulations 1-2 and 5-13 (described in the Examples). The dissolution values represent the lowest value of all the calculated similarity factors for the above-described Formulations.

**Table 3**

| Percentage (%) dissolved | Time |
|---|---|
| Less than or equal to about 70% | At about thirty (30) minutes |
| Less than or equal to about 80% | At about sixty (60) minutes |
| Less than or equal to about 90% | At about ninety (90) minutes |

**Table 4**

| |
|---|
| At least about 0.9% at about thirty (30) minutes and less than or equal to about 70% at about thirty (30) minutes |
| At least about 7.0% at about sixty (60) minutes and less than or equal to about 80% at about sixty (60) minutes |
| At least about 0.9% at about thirty (30) minutes, less than or equal to about 70% at about thirty (30) minutes, at least about 7.0% at about sixty (60) minutes and less than or equal to about 80% about sixty (60) minutes |
| At least about 0.9% at about thirty (30) minutes, less than or equal to about 70% at about thirty (30) minutes, at least about 7.0% at about sixty (60) minutes, less than or equal to about 80% at about sixty (60) minutes and less than or equal to about 90% at about ninety (90) minutes |

The dosage forms described above, after administration to a human subject under fasting conditions, all have a $C_{max}$ that does not exceed 125% of the $C_{max}$ of the reference pharmaceutical composition. Preferably, the dosage forms described above, after administration to a human subject under fasting conditions, all have a $C_{max}$ that is less than the $C_{max}$ of the reference pharmaceutical composition. Additionally, the dosage forms described above can have a $C_{max}$ that is about 125% of the $C_{max}$ of the reference pharmaceutical composition, a $C_{max}$ that is about 120% of the $C_{max}$ of the reference pharmaceutical composition, a $C_{max}$ that is about 115% of the $C_{max}$ of the reference pharmaceutical composition, a $C_{max}$ that is about 110% of the $C_{max}$ of the reference pharmaceutical composition, a $C_{max}$ that is about 105% of the $C_{max}$ of the reference pharmaceutical composition or a $C_{max}$ that is about 100% of the $C_{max}$ of the reference pharmaceutical composition. Moreover, the dosage forms described above have a $C_{max}$ that is about 95% of the $C_{max}$ of the reference pharmaceutical composition, a $C_{max}$ that is about 90% of the $C_{max}$ of the reference pharmaceutical composition, a $C_{max}$ that is about 85% of the $C_{max}$ of the reference pharmaceutical composition, or a $C_{max}$ that is about 80% of the $C_{max}$ of the reference pharmaceutical composition. Thereupon, the dosage forms described herein would be expected to provide a safety profile that is comparable to or better than the reference pharmaceutical composition.

Furthermore, the dosage forms described above, after administration to a human subject under fasting conditions, exhibit an AUC that is at least 65% of the AUC of the reference pharmaceutical composition. More specifically, the dosage forms described above, after administration to a human subject under fasting conditions, exhibit an AUC that is at least 70%, of the AUC of the reference pharmaceutical composition, exhibit an AUC that is at least 75% of the AUC of the reference pharmaceutical composition, at least 80% of the AUC of the reference pharmaceutical composition, at least 85% of the AUC of the reference pharmaceutical composition, at least 90% of the AUC of the reference pharmaceutical composition, at least 95% of the AUC of the reference pharmaceutical composition, at least 100% of the AUC of the reference pharmaceutical composition, at least 105% of the AUC of the reference pharmaceutical composition, at least 110% of the AUC of the reference pharmaceutical composition, at least 115% of the AUC of the reference pharmaceutical composition, at least 120% of the AUC of the reference pharmaceutical composition; or at least 125% of the AUC of the reference pharmaceutical composition.

In a second aspect, the present disclosure relates to solid dosage forms where the release characteristics of at least one active agent from said solid dosage forms follows a square root of time (t) profile in an in *vitro* dissolution at a single pH. Dissolution profiles that follow the square root of time are known in the art and are described, for example in T. Higuchi, J. Pharm. Sci., 52:1145 (1963), Peppas et al., Pharm. Acta. Helv., 60:110-111 (1985); and J.L. Ford et al., Int. J. Pharm., 71:95-104 (1991)). The square root of time profile used herein has the formula:

$$\% \text{ Dissolved} = A + B(t^n)$$

where A is the estimated Y-intercept in units of % dissolution. B is a constant related to the rate of dissolution. A and B are characteristics of a specific solid dosage form containing at least one active agent, t is time (in hours), n is a diffusional exponent. For the solid dosage forms of the present disclosure, n is from about 0.4 to about 0.8, preferably from about 0.4 to about 0.7.

The percentage (%) of the active agent of the dosage form dissolved in an *in vitro* dissolution at a single pH is as shown below in Table 5.

**Table 5**

| Percentage (%) dissolved | Time |
|---|---|
| At least about 15.0% but less than or equal to about 71.0% | At about 0.5 hours (30 minutes) |
| At least about 40.05 but less than or equal to about 81.0% | At about 1.0 hour |

The dissolution of the active agent at a single pH in said *in vitro* dissolution is measured at a minimum of three (3) time points that are selected before the percentage dissolved is about 80%. Preferably, these measurements are made in replicates of at least 3 (although a higher number of replicates can be used) and the mean (also known as the average) from these measurements used in the square root of time formula. The dosage forms described above, after administration to a human subject under fasting conditions, all have a $C_{max}$ that does not exceed 125% of the $C_{max}$ of the reference pharmaceutical composition. Preferably, the dosage forms described above, after administration to a human subject under fasting conditions, all have a $C_{max}$ that is less than the $C_{max}$ of the reference pharmaceutical composition. Additionally, the dosage forms described above can have a $C_{max}$ that is about 125% of the $C_{max}$ of the reference pharmaceutical composition, a $C_{max}$ that is about 120% of the $C_{max}$ of the reference pharmaceutical composition, a $C_{max}$ that is about 115% of the $C_{max}$ of the reference pharmaceutical composition, a $C_{max}$ that is about 110% of the $C_{max}$ of the reference pharmaceutical composition, a $C_{max}$ that is about 105% of the $C_{max}$ of the reference pharmaceutical composition or a $C_{max}$ that is about 100% of the $C_{max}$ of the reference pharmaceutical composition. Moreover, the dosage forms described above have a $C_{max}$ that is about 95% of the $C_{max}$ of the reference pharmaceutical composition, a $C_{max}$ that is about 90% of the $C_{max}$ of the reference pharmaceutical composition, a $C_{max}$ that is about 85% of the $C_{max}$ of the reference pharmaceutical composition, or a $C_{max}$ that is about 80% of the $C_{max}$ of the reference pharmaceutical composition. Thereupon, the dosage forms described herein would be expected to provide a safety profile that is comparable to or better than the reference pharmaceutical composition.

Furthermore, the dosage forms described above, after administration to a human subject under fasting conditions, exhibit an AUC that is at least 65% of the AUC of the reference pharmaceutical composition. More specifically, the dosage forms described above, after administration to a human subject under fasting conditions, exhibit an AUC that is at least 70% of the AUC of the reference pharmaceutical composition, exhibit an AUC that is at least 75% of the AUC of the reference pharmaceutical composition, exhibit an AUC that is at least 80% of the AUC of the reference pharmaceutical composition, at least 85% of the AUC of the reference pharmaceutical composition, at least 90% of the AUC of the reference pharmaceutical composition, at least 95% of the AUC of the reference pharmaceutical composition, at least 100% of the AUC of the reference pharmaceutical composition, at least 105% of the AUC of the reference pharmaceutical composition, at least 110% of the AUC of the reference pharmaceutical composition, at least 115% of the AUC of the reference pharmaceutical composition, at least 120% of the AUC of the reference pharmaceutical composition; or at least 125% of the AUC of the reference pharmaceutical composition.

In a third aspect, the present relates to dosage forms comprising at least one active agent, where the percentage (%) of the active agent of the dosage form dissolved in an *in vitro* dissolution at a single pH is as shown below in Table 6. The dissolution values shown below in Table 6 were determined using a similarity factor. The lower limits of the dissolution values shown in Table 6 were determined based on the similarity factor calculations using Formulations 1-2 and 5-13. The dissolution values represent the lowest value of all the calculated similarity factors for the above-described Formulations. The upper limits of the dissolution values shown in Table 6 were determined based on the similarity factor calculations using Neat Drug. The dissolution values represent the lower value of the calculated similarity factors for the Neat Drug.

**Table 6**

| Percentage (%) dissolved | Time |
|---|---|
| Less than or equal to 56.0% | About 0.25 hours (15 minutes) |

(continued)

| Percentage (%) dissolved | Time |
|---|---|
| At least about 0.9% but less than or equal to about 62.0% | About 0.5 hours (30 minutes) |
| Less than or equal to 66.0% | About 0.75 hours (45 minutes) |
| At least about 7.0% but less than or equal to about 71.0% | About one (1) hour (60 minutes) |
| Less than or equal to 79% | About 1.5 hours (90 minutes) |

The dosage forms described above, after administration to a human subject under fasting conditions, all have a $C_{max}$ that does not exceed 125% of the $C_{max}$ of the reference pharmaceutical composition. Preferably, the dosage forms described above, after administration to a human subject under fasting conditions, all have a $C_{max}$ that is less than the $C_{max}$ of the reference pharmaceutical composition. Additionally, the dosage forms described above can have a $C_{max}$ that is about 125% of the $C_{max}$ of the reference pharmaceutical composition, a $C_{max}$ that is about 120% of the $C_{max}$ of the reference pharmaceutical composition, a $C_{max}$ that is about 115% of the $C_{max}$ of the reference pharmaceutical composition, a $C_{max}$ that is about 110% of the $C_{max}$ of the reference pharmaceutical composition, a $C_{max}$ that is about 105% of the $C_{max}$ of the reference pharmaceutical composition or a $C_{max}$ that is about 100% of the $C_{max}$ of the reference pharmaceutical composition. Moreover, the dosage forms described above have a $C_{max}$ that is about 95% of the $C_{max}$ of the reference pharmaceutical composition, a $C_{max}$ that is about 90% of the $C_{max}$ of the reference pharmaceutical composition, a $C_{max}$ that is about 85% of the $C_{max}$ of the reference pharmaceutical composition, or a $C_{max}$ that is about 80% of the $C_{max}$ of the reference pharmaceutical composition. Thereupon, the dosage forms described herein would be expected to provide a safety profile that is comparable to or better than the reference pharmaceutical composition.

Furthermore, the dosage forms described above, after administration to a human subject under fasting conditions, exhibit an AUC that is at least 65% of the AUC of the reference pharmaceutical composition. More specifically, the dosage forms described above, after administration to a human subject under fasting conditions, exhibit an AUC that is at least 70%, of the AUC of the reference pharmaceutical composition, exhibit an AUC that is at least 75% of the AUC of the reference pharmaceutical composition, at least 80% of the AUC of the reference pharmaceutical composition, at least 85% of the AUC of the reference pharmaceutical composition, at least 90% of the AUC of the reference pharmaceutical composition, at least 95% of the AUC of the reference pharmaceutical composition, at least 100% of the AUC of the reference pharmaceutical composition, at least 105% of the AUC of the reference pharmaceutical composition, at least 110% of the AUC of the reference pharmaceutical composition, at least 115% of the AUC of the reference pharmaceutical composition, at least 120% of the AUC of the reference pharmaceutical composition; or at least 125% of the AUC of the reference pharmaceutical composition.

In a fourth aspect, the present relates to dosage forms comprising at least one active agent, where the percentage (%) of the active agent of the dosage form dissolved in an in *vitro* dissolution at a single pH is as shown below in Tables 7, 8, 9, 10, 11 and 12. The dissolution values shown below in Tables 7, 8, 9, 10, 11 and 12 were determined using a similarity factor. The lower limits of the dissolution values shown in Tables 7, 8, 11 and 12 were determined based on the similarity factor calculations using Formulations 1-2 and 5-13. The dissolution values represent the lowest value of all the calculated similarity factors for the above-described Formulations. The upper limits of the dissolution values shown in Tables 9, 10, 11 and 12 were determined based on the similarity factor calculations using Formulations 1-2 and 5-13. The dissolution values represent the highest value of all the calculated similarity factors for the above-described Formulations.

Table **7**

| Percentage (%) dissolved | Time |
|---|---|
| At least about 7.0% | About one (1) hour |
| At least about 16.0% | About two (2) hours |
| At least about 24.0% | About three (3) hours |
| At least about 28.0% | About three and one half (3.5) hours |
| At least about 29.0% | About four (4) hours |
| At least about 31.0% | At about five (5) hours |
| At least about 32.0% | At about six (6) hours |

(continued)

| Percentage (%) dissolved | Time |
|---|---|
| At least about 35.0% | At about eight (8) hours |
| At least about 37.0% | At about ten (10) hours |

### Table 8

| |
|---|
| At least about 7.0% at about 1 hour and at least about 16.0% at about 2 hours |
| At least about 7.0% at about 1 hour, at least about 16.0% at about 2 hours and at least about 24.0% at about 3 hours |
| At least about 7.0% at about 1 hour, at least about 16.0% at about 2 hours, at least about 24.0% at about 3 hours, and at least about 28.0% at about 3.5 hours |
| At least about 7.0% at about 1 hour, at least about 16.0% at about 2 hours, at least about 24.0% at about 3 hours, at least about 28.0% at about 3.5 hours and at least about 29.0% at about 4 hours |
| At least about 7.0% at about 1 hour, at least about 16.0% at about 2 hours, at least about 24.0% at about 3 hours, at least about 28.0% at about 3.5 hours, at least about 29.0% at about 4 hours and at least about 31.0% at about 5 hours |
| At least about 7.0% at about 1 hour, at least about 16.0% at about 2 hours, at least about 24.0% at about 3 hours, at least about 28.0% at about 3.5 hours, at least about 29.0% at about 4 hours, at least about 31.0% at about 5 hours, and at least about 32.0% at about 6 hours |
| At least about 7.0% at about 1 hour, at least about 16.0% at about 2 hours, at least about 24.0% at about 3 hours, at least about 28.0% at about 3.5 hours, at least about 29.0% at about 4 hours, at least about 31.0% at about 5 hours, at least about 32.0% at about 6 hours, and at least about 35.0% at about 8 hours |
| At least about 7.0% at about 1 hour, at least about 16.0% at about 2 hours, at least about 24.0% at about 3 hours, at least about 28.0% at about 3.5 hours, at least about 29.0% at about 4 hours, at least about 31.0% at about 5 hours, at least about 32.0% at about 6 hours, at least about 35.0% at about 8 hours and at least about 37.0% at about 10 hours |

### Table 9

| Percentage (%) dissolved | Time |
|---|---|
| Less than or equal to about 41.0% | At about one (1) hour |
| Less than or equal to about 79.0% | At about two (2) hours |

### Table 10

| |
|---|
| Less than or equal to about 41.0% at about 1 hour and less than or equal to about 79.0% at about 2 hours |

### Table 11

| Percentage (%) dissolved | Time |
|---|---|
| At least about 7.0% but less than or equal to about 41.0% | About one (1) hour |
| At least about 16.0% but less than or equal to about 79.0% | About two (2) hours |
| At least about 24.0% but less than or equal to about 100.0% | About three (3) hours |
| At least about 28.0% but less than or equal to about 100.0% | About three and one half (3.5) hours |
| At least about 29.0% but less than or equal to about 100.0% | About four (4) hours |
| At least about 31.0% but less than or equal to about 100.0% | At about five (5) hours |
| At least about 32.0 but less than or equal to about 100.0% | At about six (6) hours |

(continued)

| Percentage (%) dissolved | Time |
|---|---|
| At least about 35.0% but less than or equal to about 100.0% | At about eight (8) hours |
| At least about 37.0% but less than or equal to about 100.0% | At about ten (10) hours |

**Table 12**

| |
|---|
| At least about 7.0% but less than or equal to about 41.0% at about 1 hour and at least about 16.0% but less than or equal to about 79.0% at about 2 hours |
| At least about 7.0% but less than or equal to about 41.0% at about 1 hour, at least about 16.0% but less than or equal to about 79.0% at about 2 hours and at least about 24.0% but less than or equal to about 100.0% at 3 hours |
| At least about 7.0% but less than or equal to about 41.0% at about 1 hour, at least about 16.0% but less than or equal to about 79.0% at about 2 hours, at least about 24.0% but less than or equal to about 100.0% at about 3 hours, and at least about 28.0% but less than or equal to about 100.0% at about 3.5 hours |
| At least about 7.0% but less than or equal to about 41.0% at about 1 hour, at least about 16.0% but less than or equal to about 79.0% at about 2 hours, at least about 24.0% but less than or equal to about 100.0% at about 3 hours, at least about 28.0% but less than or equal to about 100.0% at about 3.5 hours and at least 29.0% but less than or equal to about 100.0% at about 4 hours |
| At least about 7.0% but less than or equal to about 41.0% at about 1 hour, at least about 16.0% but less than or equal to about 79.0% at about 2 hours, at least about 24.0% but less than or equal to about 100.0% at about 3 hours, at least about 28.0% but less than or equal to about 100.0% at about 3.5 hours, at least 29.0% but less than or equal to about 100.0% at about 4 hours and at least about 31.0% but less than or equal to about 100.0% at about 5 hours |
| At least about 7.0% but less than or equal to about 41.0% at about 1 hour, at least about 16.0% but less than or equal to about 79.0% at about 2 hours, at least about 24.0% but less than or equal to about 100.0% at about 3 hours, at least about 28.0% but less than or equal to about 100.0% at about 3.5 hours, at least 29.0% but less than or equal to about 100.0% at about 4 hours, at least about 31.0% but less than or equal to about 100.0% at about 5 hours, and at least about 32.0% but less than or equal to about 100.0% at about 6 hours |
| At least about 7.0% but less than or equal to about 41.0% at about 1 hour, at least about 16.0% but less than or equal to about 79.0% at about 2 hours, at least about 24.0% but less than or equal to about 100.0% at about 3 hours, at least about 28.0% but less than or equal to about 100.0% at about 3.5 hours, at least 29.0% but less than or equal to about 100.0% at about 4 hours, at least about 31.0% but less than or equal to about 100.0% at about 5 hours, at least about 32.0% but less than or equal to about 100.0% at about 6 hours, and at least about 35.0% but less than or equal to about 100.0% at about 8 hours |
| At least about 7.0% but less than or equal to about 41.0% at about 1 hour, at least about 16.0% but less than or equal to about 79.0% at about 2 hours, at least about 24.0% but less than or equal to about 100.0% at about 3 hours, at least about 28.0% but less than or equal to about 100.0% at about 3.5 hours, at least 29.0% but less than or equal to about 100.0% at about 4 hours, at least about 31.0% but less than or equal to about 100.0% at about 5 hours, at least about 32.0% but less than or equal to about 100.0% at about 6 hours, at least about 35.0% but less than or equal to about 100.0% at about 8 hours and at least about 37.0% but less than or equal to about 100.0% at about 10 hours |

The dosage forms described above, after administration to a human subject under fasting conditions, all have a $C_{max}$ that does not exceed 125% of the $C_{max}$ of the reference pharmaceutical composition. Preferably, the dosage forms described above, after administration to a human subject under fasting conditions, all have a $C_{max}$ that is less than the $C_{max}$ of the reference pharmaceutical composition. Additionally, the dosage forms described above can have a $C_{max}$ that is about 125% of the $C_{max}$ of the reference pharmaceutical composition, a $C_{max}$ that is about 120% of the $C_{max}$ of the reference pharmaceutical composition, a $C_{max}$ that is about 115% of the $C_{max}$ of the reference pharmaceutical composition, a $C_{max}$ that is about 110% of the $C_{max}$ of the reference pharmaceutical composition, a $C_{max}$ that is about 105% of the $C_{max}$ of the reference pharmaceutical composition or a $C_{max}$ that is about 100% of the $C_{max}$ of the reference pharmaceutical composition. Moreover, the dosage forms described above have a $C_{max}$ that is about 95% of the $C_{max}$ of the reference pharmaceutical composition, a $C_{max}$ that is about 90% of the $C_{max}$ of the reference pharmaceutical

composition, a $C_{max}$ that is about 85% of the $C_{max}$ of the reference pharmaceutical composition, or a $C_{max}$ that is about 80% of the $C_{max}$ of the reference pharmaceutical composition. Thereupon, the dosage forms described herein would be expected to provide a safety profile that is comparable to or better than the reference pharmaceutical composition.

Furthermore, the dosage forms described above, after administration to a human subject under fasting conditions, exhibit an AUC that is at least 65% of the AUC of the reference pharmaceutical composition. More specifically, the dosage forms described above, after administration to a human subject under fasting conditions, exhibit an AUC that is at least 70%, of the AUC of the reference pharmaceutical composition, exhibit an AUC that is at least 75% of the AUC of the reference pharmaceutical composition, at least 80% of the AUC of the reference pharmaceutical composition, at least 85% of the AUC of the reference pharmaceutical composition, at least 90% of the AUC of the reference pharmaceutical composition, at least 95% of the AUC of the reference pharmaceutical composition, at least 100% of the AUC of the reference pharmaceutical composition, at least 105% of the AUC of the reference pharmaceutical composition, at least 110% of the AUC of the reference pharmaceutical composition, at least 115% of the AUC of the reference pharmaceutical composition, at least 120% of the AUC of the reference pharmaceutical composition; or at least 125% of the AUC of the reference pharmaceutical composition.

In a fifth aspect, the present relates to dosage forms comprising at least one active agent, where the percentage (%) of the active agent of the dosage form dissolved in an *in vitro* dissolution at a single pH is as shown below in Tables 13, 14, 15 and 16. The dissolution values shown below in Tables 13, 14, 15 and 16 were determined using a similarity factor. The lower limits of the dissolution values shown in Tables 13 and 14 were determined based on the similarity factor calculations using Formulations 1, 5, 8 and 9-13. The dissolution values represent the lowest value of all the calculated similarity factors for the above-described Formulations. The upper limits of the dissolution values shown in Tables 15 and 16 were determined based on the similarity factor calculations using Formulations 1, 5, 8 and 9-13. The dissolution values represent the highest value of all the calculated similarity factors for the above-described Formulations.

**Table 13**

| Percentage (%) dissolved | Time |
|---|---|
| At least about 9.0% | About one (1) hour |
| At least about 21.0% | About two (2) hours |
| At least about 34.0% | About three (3) hours |
| At least about 39.0% | About three and one half (3.5) hours |
| At least about 44.0% | About four (4) hours |
| At least about 49.0% | At about five (5) hours |
| At least about 54.0% | At about six (6) hours |
| At least about 60.0% | At about eight (8) hours |
| At least about 67.0% | At about ten (10) hours |

**Table 14**

| |
|---|
| At least about 9.0% at about 1 hour and at least about 21.0% at about 2 hours |
| At least about 9.0% at about 1 hour, at least about 21.0% at about 2 hours and at least about 34.0% at about 3 hours |
| At least about 9.0% at about 1 hour, at least about 21.0% at about 2 hours, at least about 34.0% at about 3 hours, and at least about 67.0% at about 3.5 hours |
| At least about 9.0% at about 1 hour, at least about 21.0% at about 2 hours, at least about 34.0% at about 3 hours, at least about 67.0% at about 3.5 hours and at least about 44.0% at about 4 hours |
| At least about 9.0% at about 1 hour, at least about 21.0% at about 2 hours, at least about 34.0% at about 3 hours, at least about 67.0% at about 3.5 hours, at least about 44.0% at about 4 hours and at least about 49.0% at about 5 hours |
| At least about 9.0% at about 1 hour, at least about 21.0% at about 2 hours, at least about 34.0% at about 3 hours, at least about 67.0% at about 3.5 hours, at least about 44.0% at about 4 hours, at least about 49.0% at about 5 hours, and at least about 54.0% at about 6 hours |

(continued)

| |
|---|
| At least about 9.0% at about 1 hour, at least about 21.0% at about 2 hours, at least about 34.0% at about 3 hours, at least about 67.0% at about 3.5 hours, at least about 44.0% at about 4 hours, at least about 49.0% at about 5 hours, at least about 54.0% at about 6 hours, and at least about 60.0% at about 8 hours |
| At least about 9.0% at about 1 hour, at least about 21.0% at about 2 hours, at least about 34.0% at about 3 hours, at least about 67.0% at about 3.5 hours, at least about 44.0% at about 4 hours, at least about 49.0% at about 5 hours, at least about 54.0% at about 6 hours, at least about 60.0% at about 8 hours and at least about 67.0% at about 10 hours |

**Table 15**

| Percentage (%) dissolved | Time |
|---|---|
| At least about 9.0% but less than or equal to about 41.0% | About one (1) hour |
| At least about 21.0% but less than or equal to about 79.0% | About two (2) hours |
| At least about 34.0% but less than or equal to about 100.0% | About three (3) hours |
| At least about 39.0% but less than or equal to about 100.0% | About three and one half (3.5) hours |
| At least about 44.0% but less than or equal to about 100.0% | About four (4) hours |
| At least about 49.0% but less than or equal to about 100.0% | At about five (5) hours |
| At least about 54.0 but less than or equal to about 100.0% | At about six (6) hours |
| At least about 60.0% but less than or equal to about 100.0% | At about eight (8) hours |
| At least about 67.0% but less than or equal to about 100.0% | At about ten (10) hours |

**Table 16**

| |
|---|
| At least about 9.0% but less than or equal to about 41.0% at about 1 hour and at least about 21.0% but less than or equal to about 79.0% at about 2 hours |
| At least about 9.0% but less than or equal to about 41.0% at about 1 hour, at least about 21.0% but less than or equal to about 79.0% at about 2 hours and at least about 34.0% but less than or equal to about 100.0% at about 3 hours |
| At least about 9.0% but less than or equal to about 41.0% at about 1 hour, at least about 21.0% but less than or equal to about 79.0% at about 2 hours, at least about 34.0% but less than or equal to about 100.0% at about 3 hours, and at least about 39.0% but less than or equal to about 100.0% at about 3.5 hours |
| At least about 9.0% but less than or equal to about 41.0% at about 1 hour, at least about 21.0% but less than or equal to about 79.0% at about 2 hours, at least about 34.0% but less than or equal to about 100.0% at about 3 hours, at least about 39.0% but less than or equal to about 100.0% at about 3.5 hours and at least about 44.0% but less than or equal to about 100.0% at about 4 hours |
| At least about 9.0% but less than or equal to about 41.0% at about 1 hour, at least about 21.0% but less than or equal to about 79.0% at about 2 hours, at least about 34.0% but less than or equal to about 100.0% at about 3 hours, at least about 39.0% but less than or equal to about 100.0% at about 3.5 hours, at least about 44.0% but less than or equal to about 100.0% at 4 hours and at least about 49.0% but less than or equal to about 100.0% at about 5 hours |
| At least about 9.0% but less than or equal to about 41.0% at about 1 hour, at least about 21.0% but less than or equal to about 79.0% at 2 hours, at least about 34.0% but less than or equal to about 100.0% at about 3 hours, at least about 39.0% but less than or equal to about 100.0% at about 3.5 hours, at least about 44.0% but less than or equal to about 100.0% at about 4 hours, at least about 49.0% but less than or equal to about 100.0% at about 5 hours, and at least about 54.0% but less than or equal to about 100.0% at about 6 hours |

(continued)

| At least about 9.0% but less than or equal to about 41.0% at about 1 hour, at least about 21.0% but less than or equal to about 79.0% at about 2 hours, at least about 34.0% but less than or equal to about 100.0% at about 3 hours, at least about 39.0% but less than or equal to about 100.0% at about 3.5 hours, at least about 44.0% but less than or equal to about 100.0% at about 4 hours, at least about 49.0% but less than or equal to about 100.0% at about 5 hours, at least about 54.0% but less than or equal to about 100.0% at about 6 hours, and at least about 60.0% but less than or equal to about 100.0% at about 8 hours |
| At least about 9.0% but less than or equal to about 41.0% at about 1 hour, at least about 21.0% but less than or equal to about 79.0% at about 2 hours, at least about 34.0% but less than or equal to about 100.0% at about 3 hours, at least about 39.0% but less than or equal to about 100.0% at about 3.5 hours, at least about 44.0% but less than or equal to about 100.0% at about 4 hours, at least about 49.0% but less than or equal to about 100.0% at about 5 hours, at least about 54.0% but less than or equal to about 100.0% at about 6 hours, at least about 60.0% but less than or equal to about 100.0% at about 8 hours and at least about 67.0% but less than or equal to about 100.0% at about 10 hours |

The dosage forms having the dissolution values shown above, after administration to a human subject under fasting conditions exhibit a similar AUC (namely, they are bioequivalent) to the AUC of the reference pharmaceutical composition. Thereupon, the dosage forms described herein would be expected to provide comparable efficacy to the reference pharmaceutical composition.

Furthermore, the dosage forms described above, after administration to a human subject under fasting conditions, exhibit an AUC that is at least 65% of the AUC of the reference pharmaceutical composition. More specifically, the dosage forms described above, after administration to a human subject under fasting conditions, exhibit an AUC that is at least 70%, of the AUC of the reference pharmaceutical composition, exhibit an AUC that is at least 75% of the AUC of the reference pharmaceutical composition, at least 80% of the AUC of the reference pharmaceutical composition, at least 85% of the AUC of the reference pharmaceutical composition, at least 90% of the AUC of the reference pharmaceutical composition, at least 95% of the AUC of the reference pharmaceutical composition, at least 100% of the AUC of the reference pharmaceutical composition, at least 105% of the AUC of the reference pharmaceutical composition, at least 110% of the AUC of the reference pharmaceutical composition, at least 115% of the AUC of the reference pharmaceutical composition, at least 120% of the AUC of the reference pharmaceutical composition; or at least 125% of the AUC of the reference pharmaceutical composition.

The dosage forms described above, after administration to a human subject under fasting conditions, all have a $C_{max}$ that does not exceed 125% of the $C_{max}$ of the reference pharmaceutical composition. Preferably, the dosage forms described above, after administration to a human subject under fasting conditions, all have a $C_{max}$ that is less than the $C_{max}$ of the reference pharmaceutical composition. Additionally, the dosage forms described above can have a $C_{max}$ that is about 125% of the $C_{max}$ of the reference pharmaceutical composition, a $C_{max}$ that is about 120% of the $C_{max}$ of the reference pharmaceutical composition, a $C_{max}$ that is about 115% of the $C_{max}$ of the reference pharmaceutical composition, a $C_{max}$ that is about 110% of the $C_{max}$ of the reference pharmaceutical composition, a $C_{max}$ that is about 105% of the $C_{max}$ of the reference pharmaceutical composition or a $C_{max}$ that is about 100% of the $C_{max}$ of the reference pharmaceutical composition. Moreover, the dosage forms described above have a $C_{max}$ that is about 95% of the $C_{max}$ of the reference pharmaceutical composition, a $C_{max}$ that is about 90% $C_{max}$ of the reference pharmaceutical composition, a $C_{max}$ that is about 85% of the $C_{max}$ of the reference pharmaceutical composition, or a $C_{max}$ that is about 80% of the $C_{max}$ of the reference pharmaceutical composition. Thereupon, the dosage forms described herein would be expected to provide a safety profile that is comparable to or better than the reference pharmaceutical composition.

B. Lack of Significant Food Effect on Oral Administration

The present invention also relates to solid dosage forms containing at least one active agent that lack a significant food effect upon oral administration of said dosage form to one or more subjects in need of treatment thereof. As used herein, the term "lacks a significant food effect" means (a) that when a solid dosage form of the present invention containing an active agent is orally administered to a human subject under fed conditions that said dosage form exhibits an AUC that is similar to (or does not differ substantially from) the AUC of said dosage form after oral administration to a human subject under fasting conditions (meaning, that the AUC of the dosage form of the present invention after oral administration to a human subject under fed conditions is not affected by food); or (b) that when the $C_{max}$ of the solid dosage form of the present invention (hereinafter "$C_{max}$ P.I.") after oral administration to a human subject under fed or fasting conditions is divided by the $C_{max}$ of the reference pharmaceutical composition (hereinafter "$C_{max}$ R.P.C."), that the resulting ratio is less than 1.25 (namely, [$C_{max}$P.I. (fed or fasted) / $C_{max}$ R.P.C. (low fat fed conditions)] < 1.25).

Preferably, the solid dosage forms of the present disclosure, upon oral administration to a human subject in a fed and fasted state, has an AUC(fed)/AUC(fasted) that is between 0.70 and 1.43 or more preferably, between 0.80 and 1.25.

In one aspect, the present disclosure relates to dosage forms comprising an active agent, where said dosage form exhibits a $\Delta diss_{2.0hr}$ of between -10.0 to 17.0, preferably between -10.0 to 9.0, more preferably between -10.0 to 2.0. Dosage forms exhibiting a $\Delta diss_{2.0hr}$ of between -10.0 to 17.0, preferably -10 to 9.0, more preferably, -10.0 to 2.0 (hereinafter referred to as the "$\Delta$ dissolution ranges") have been found to lack a significant food effect upon oral administration to a human subject under fed conditions. Not only do dosage forms exhibiting the $\Delta$ dissolution range exhibit a lack of a significant food effect upon oral administration, but the hereinbefore described correlation, namely, the difference in release as measured using $\Delta diss_t$ provides a predictive model that can be used to describe the relationship between an *in vitro* property (namely, dissolution) of the solid dosage form and an *in vivo* property, namely, food effect. This model can be used to aid in the development of new solid dosages comprising the active ingredient. Specifically, this model allows a formulator to perform a relatively quick *in vitro* dissolution test to determine whether or not a newly developed dosage form containing the active ingredient is likely to exhibit a significant food effect upon oral administration to a subject under fed conditions without the need to conduct more expensive *in vivo* (namely human) tests. Using this model, dosage forms identified as likely not to exhibit a significant food effect upon oral administration can be targeted for further product development while those dosage forms identified as likely to exhibit a significant food effect can be discontinued from any further development.

The above-described model was developed by first determining $\Delta diss_{2.0hr}$ for each of seven dosage forms containing at least one active agent (namely, dosage forms 1, 2, 5, 6, 7, 8 and 10 which are described in more detail in the Examples). The $\Delta diss_{2.0hr}$ obtained for each dosage form was considered to be a predictor of an *in vivo* food effect with respect to $C_{max}$.

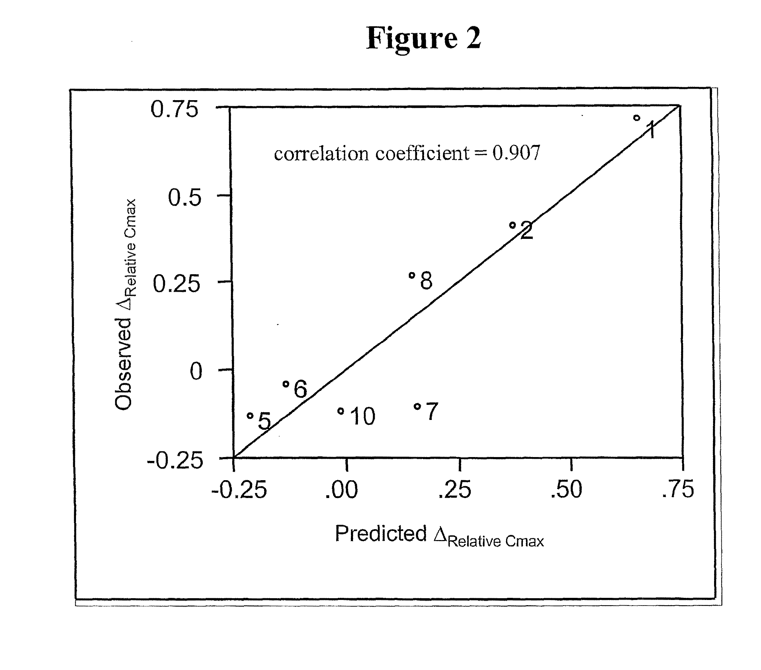

In addition to the $\Delta diss_{2.0hr}$, the Observed $\Delta$Relative $C_{max}$ was determined for each of the seven dosage forms described above. The Observed $\Delta$Relative $C_{max}$ values obtained for each dosage form were then regressed on $\Delta diss_{2.0hr}$ using simple linear regression to obtain the least squares regression prediction equation involving only a slope and intercept. This regression prediction equation was used to obtain the predicted $\Delta$Relative $C_{max}$ (hereinafter "Predicted $\Delta$Relative $C_{max}$"). The minimum and maximum Predicted $\Delta$Relative $C_{max}$ values were taken as the acceptable range for Predicted $\Delta$Relative $C_{max}$. The associated acceptable ranges for the $\Delta diss_{2.0hr}$ (namely, the $\Delta$ dissolution range described above) were obtained by solving the regression prediction equation for $\Delta diss_{2.0hr}$ at the minimum and maximum range for the Predicted $\Delta$Relative $C_{max}$ (See Figure 2).

In another aspect, the present disclosure relates to dosage forms comprising an active agent, where said dosage form has a Predicted $\Delta$Relative $C_{max}$ of -0.2 to +0.8, preferably from -0.2 to +0.2 (hereinafter referred to as the "Predicted $\Delta$Relative $C_{max}$ range"). In order to determine whether a solid dosage form has a Predicted $\Delta$Relative $C_{max}$ of -0.2 to +0.8, the following formula is used:

$$\text{Predicted } \Delta\text{Relative } C_{max} = 0.096 - 0.035\Delta diss_{0.5h} + 0.082\Delta diss_{1.0h} + 0.037\Delta diss_{2.0h}$$

Dosage forms having a Predicted $\Delta$Relative $C_{max}$ that fall within the above-described Predicted $\Delta$Relative $C_{max}$ range (namely, -0.2 to +0.8, preferably -0.2 to +0.2), lack a significant food effect upon oral administration to a subject under fed or fasted conditions. Not only do dosage forms having a value within the above Predicted $\Delta$Relative $C_{max}$ range exhibit a lack of a significant food effect upon oral administration, but the hereinbefore described correlation provides a predictive model that can be used to describe the relationship between the *in vitro* property (namely, dissolution) of the solid dosage form and an *in vivo* property, namely, food effect. This model can be used to aid in the development of new solid dosages comprising the active ingredient. Specifically, this model allows a formulator to perform a relatively quick *in vitro* dissolution test to determine whether or not a newly developed dosage form containing the active ingredient is likely to exhibit a significant food effect upon oral administration to a subject under fed conditions without the need to conduct more expensive *in vivo* (namely human) tests. Using this model, dosage forms identified as likely not to exhibit a significant food effect upon oral administration can be targeted for further product development while those dosage forms identified as likely to exhibit a significant food effect can be discontinued from any further development.

The above-described model was developed by first determining the $\Delta diss_{0.5hr}$, $\Delta diss_{1.0hr}$ and $\Delta diss_{2.0hr}$ for seven formulations (namely, dosage forms 1, 2, 5, 6, 7, 8 and 10 which are described in more detail in the Examples). The $\Delta diss_{0.5hr}$, $\Delta diss_{1.0hr}$ and $\Delta diss_{2.0hr}$ obtained for each dosage form at each of these time points were considered to be predictors of an *in vivo* food effect with respect to $C_{max}$.

In addition to $\Delta diss_{0.5hr}$, $\Delta diss_{1.0hr}$ and $\Delta diss_{2.0hr}$ described above, the Observed $\Delta$Relative $C_{max}$ was determined for each of the seven dosage forms above. The Observed $\Delta$Relative $C_{max}$ for each dosage form was then regressed on $\Delta diss_{0.5hr}$, $\Delta diss_{1.0hr}$ and $\Delta diss_{2.0hr}$ using multiple linear regression to obtain the least squares regression prediction equation. The regression prediction equation ("hereinafter "RPE") obtained was the same equation used for the Predicted

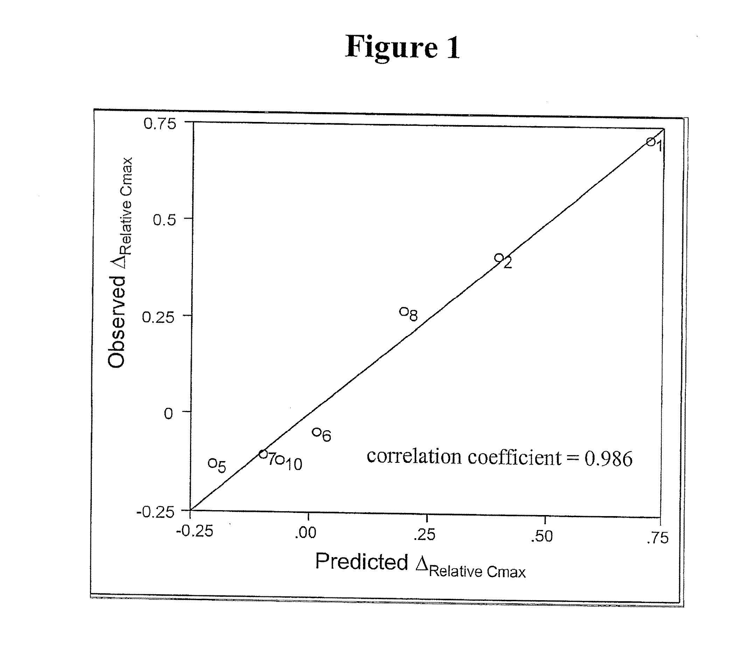

$\Delta$Relative $C_{max}$ described herein (namely, Predicted $\Delta$Relative $C_{max}$ = 0.096 - 0.035$\Delta$diss$_{0.5h}$ + 0.082$\Delta$diss$_{1.0h}$ + 0.037$\Delta$diss$_{2.0h}$). Thus, this equation was used to obtain the Predicted $\Delta$Relative $C_{max}$. The minimum and maximum Predicted $\Delta$Relative $C_{max}$ values were taken as the acceptable range for Predicted $\Delta$Relative $C_{max}$. A correlation plot of the Observed $\Delta$Relative $C_{max}$ was (on the Y axis) versus the minimum and maximum Predicted $\Delta$Relative $C_{max}$ (on the X axis) is shown in Figure 1. The correlation coefficient was determined to be 0.986.

III. Composition and Methods for Making the Dosage Forms of the Present Invention

The benefits of the dosage forms of the present invention can be obtained with any dosage form that provides for a modified release of the active agent. Examples of such dosage forms that can be used include, but are not limited to, matrix systems, membrane controlled systems (which are also referred to as "reservoir systems"), pulse release systems or osmotic pumps. Each of these systems is described in greater detail herein. A detailed discussion of such dosage forms may also be found in: (i) Handbook of Pharmaceutical Controlled Release Technology, ed. D. L. Wise, et al., Marcel Dekker, Inc., New York, New York (2000), and (ii) and Treatise on Controlled Drug Delivery, Fundamentals, Optimization, and Applications, ed. A. Kydonieus, Marcel Dekker, Inc., New York, New York (1992).

Matrix systems are well known to those skilled in the art. For the dosage forms of the present invention, at least one active agent is homogenously dispersed in at least one rate-controlling mechanism and optionally, with at least one pharmaceutically acceptable excipient. This admixture can be made into a dosage form such as, but not limited to, a powder, a granule, bead, pellet, particulate, a tablet, a mini tablet or an agglomerate. The present invention also contemplates that the dosage form, such as, but not limited to a powder, a granule, bead, pellet, particulate, a tablet, a mini tablet or an agglomerate, can be sprinkled on to food or dissolved in an appropriate drink for subject consumption. The present invention also contemplates that after said dosage form is made that it can be optionally surrounded or coated with one or more rate-controlling layers and/or one or more enteric coatings, which will be described in more detail herein.

As used herein, the term "at least one rate-controlling mechanism" refers to an agent that controls or modulates the rate of release of the active agent from the dosage form. The at least one rate-controlling mechanism generally comprises an inert, non-toxic material that is at least partially, and generally substantially completely erodible in an environment of use. Selection of materials suitable for the rate-controlling mechanism of the present invention will depend upon the desired period for the release of the active agent from the dosage form which is well known to those skilled in the art.

The rate-controlling mechanism used in a matrix dosage form can be a hydrophilic agent, hydrophobic agents or combinations thereof. Additionally, the rate-controlling mechanism may optionally include any pharmaceutically acceptable excipient that can help modulate the hydrophilicity and/or hydrophobicity of the hydrophilic and/or hydrophobic agents. Hydrophilic agents that can be used include, but are not limited to, hydroxypropyl methylcellulose, hydroxypropyl cellulose, hydroxyethyl cellulose, polyethylene oxide, polyethylene glycols ("PEG"), xanthan gum, alginates, polyvinyl pyrrolidone, starches, cross-linked homopolymers and copolymers of acrylic acid and other pharmaceutically acceptable substances with swelling and/or gel-forming properties and combinations thereof. Hydrophobic agents that can be used include, but are not limited to, waxes and water-insoluble agents. Examples of waxes that can be used include, but are not limited to, natural and synthetic waxes, such as, carnauba wax, bees wax, candelilla wax, paraffin waxes and combinations thereof. Water insoluble agents include, but are not limited to, ammoniomethacrylate copolymers (such as Eudragit@ RL100, RS 100), cellulose, ethylcellulose, cellulose acetates, cellulose acetate butyrate, cellulose acetate propionate, methacrylic ester copolymers (Eudragitg NE30D), microcrystalline cellulose and dibasic calcium phosphate and combinations thereof. Examples of a rate-controlling mechanism used in the form of a coating or membrane include, but are not limited to, ethylcellulose (such as Surelease® and Aquacoat@ ECD), ammoniomethacrylate copolymers (such as Eudragit RL30D and RS30D) and methacrylic ester copolymers (such as Eudragit@ NE30D).

One skilled in the art would be able to determine the types and amounts of pharmaceutically acceptable excipients that would be suitable and appropriate for such matrix dosage forms. Examples of pharmaceutically acceptable excipients that can be used in the dosage forms of the present invention include, but are not limited to, one or more fillers, binders, lubricants/glidants, solubility enhancing agents, suspending agents, sweetness and/or flavoring agents, preservatives, buffers, wetting agents, disintegrating agents, effervescent agents, surfactants, humectants, solution retarders, absorbents, solvents, other pharmaceutically acceptable additives and combinations thereof

Fillers that can be used in the present invention include, but are not limited to, starches, lactose, microcrystalline cellulose, sucrose, glucose, sorbitol, mannitol and combinations thereof. Examples of fillers that can be used are microcrystalline cellulose, such as Avicel® PH101 and Avicel® PH102; lactose, such as lactose monohydrate, lactose anhydrous and Pharmatose DCL21; dibasic calcium phosphate such as Emcompress®.

Binders that can be used in the present invention include, but are not limited to, celluloses such as hydroxypropyl methylcellulose, hydroxypropyl cellulose, microcrystalline cellulose, polyvinyl pyrrolidone, starches and other pharmaceutically acceptable substances with cohesive properties.

Lubricants and glidants that can be used in the present invention include, but are not limited to, colloidal silicon dioxide, such as Aerosil® 200, talc, stearic acid, magnesium stearate, calcium stearate, solid polyethylene glycols, sodium stearyl fumarate, silica gel and mixtures thereof and other substances with lubricating or gliding properties.

Solubility enhancing agents that can be used include, but are not limited to, cosolvents such as ethanol or propylene glycol, surfactants and polymeric substances such as polysorbates, polyalkylene glycols, poloxamers or polyvinylpyrrolidone, and oily fatty acids and their mono- or diglyceryl esters such as linoleic acid or glyceryl monolaurate.

Suspending agents that can be used include, but are not limited to, carboxymethylcelluose, veegum, tragacanth, bentonite, methylcellulose and polyethylene glycols.

Sweeteners that can be used in the present invention are any natural or artificial sweetner such as, but not limited to, sucrose, xylitol, sodium saccharin, cyclamate, aspartame and acesulfame. Examples of flavoring agents are Magnasweet®, bubble gum flavor, fruit flavors and the like.

Preservatives that can be used in the present invention include, but are not limited to, potassium sorbate, methylparaben, propylparaben, benzoic acid and its salts, other esters of parahydroxybenzoic acid such as butylparaben, alcohols such as ethyl or benzyl alcohol, phenolic compounds such as phenol or quaternary compounds such as benzalkonium chloride.

Suitable buffers that can be used in the present invention include, but are not limited to, phosphate, acetate, citrate, succinate and histidine buffers.

Wetting agents that can be used in the present invention include, but are not limited to, ammonium lauryl sulfate and sodium lauryl sulfate.

Suitable disintegrating agents that can be used in the present invention include, but are not limited to, cross-linked polyvinyl pyrrolidone, corn starch, potato starch, maize starch and modified starches, agar-agar, calcium carbonate, sodium carbonate, alginic acids, cross-carmellose sodium, sodium starch glycolate, microcrystalline cellulose and mixtures thereof.

Suitable effervescent agents that can be used in the present invention are effervescent couples such as, but not limited to, an organic acid and a carbonate or bicarbonate. Suitable organic acids include, but are not limited to, citric, tartaric, malic, fumaric, adipic, succinic, and alginic acids and anhydrides and acid salts. Suitable carbonates and bicarbonates include, but are not limited to, sodium carbonate, sodium bicarbonate, potassium carbonate, potassium bicarbonate, magnesium carbonate, sodium glycine carbonate, L-lysine carbonate and arginine carbonate.

The term "surfactant" is used in its conventional sense in the present invention. Any surfactant is suitable, whether it is amphoteric, non-ionic, cationic or anionic. Examples of suitable surfactants include, but are not limited to, sodium lauryl sulfate, polysorbates such as polyoxyethylene sorbitan monooleate, monolaurate, monopalmitate, monstearate or another ester of polyoxyethylene sorbitan (e.g., the commercially available Tweens®, such as, Tween® 20 and Tween® 80 (ICI Speciality Chemicals)), sodium dioctylsulfosuccinate (DOSS), lecithin, stearylic alcohol, cetostearylic alcohol, cholesterol, polyoxyethylene ricin oil, polyoxyethylene fatty acid glycerides, poloxamers (e.g., Pluronics F68® and F108®, which are block copolymers of ethylene oxide and propylene oxide); polyoxyethylene castor oil derivatives or mixtures thereof.

Examples of humectants that can be used, include, but are not limited to, glycerol, sorbitol, pentatol, polyethylene glycol or propylene glycol.

Examples of absorbents that can be used include, but are not limited to, kaolin and bentonite.

The dosage form can optionally be surrounded or coated with at least one non-rate-controlling layer. The functions of the non-rate-controlling layer include, but are not limited to, providing stability for the active agent, functioning as a process aid and/or as a cosmetic enhancement for the formulation. The non-rate-controlling layer can be formed as a single layer, coating or membrane or a plurality of single layers, coatings or membranes.

When the dosage form contains a non-rate-controlling layer, said non-rate-controlling layer can be made of one or more polymers, as well as, other ingredients known in the art, such as, but not limited to, plasticizers, pigments/opacifiers, waxes, etc. Examples of polymers that can be used include, but are not limited to, hydroxypropyl methylcellulose, hydroxypropyl cellulose, methylcellulose, polyvinyl alcohol and polyethylene glycol. Examples of plasticizers that can be used include, but limited to, polyethylene glycol(s), glycerin, triacetin, triethyl citrate, diethyl phthalate, and mineral oils. Examples of pigments/opacifiers that can be used include, but are not limited to, water soluble dyes (for example, sunset yellow, quinoline yellow, erythrosine, and tartrazine), pigments (for example, aluminum lakes, titanium oxides, iron oxides and talc), and natural products (for example, riboflavin, carotenoids, chlorophyll, anthocyanins, and carmine). Examples of a wax that can be used is a paraffin wax.

Matrix dosage forms can be prepared using standard techniques well known to those skilled in the art, direct blending, dry granulation (roller compaction), wet granulation (high shear granulation), milling or sieving, drying (if wet granulation is used), extrusion/spheronization, balling or compression, and, optionally, coating. For example, such dosage forms can be prepared by mixing at least one active agent, at least one rate-controlling mechanism, and optionally, at least one pharmaceutically acceptable excipient to obtain a powder blend. The powder blend can then be filled into a capsule or compressed into tablets. Additionally, the powder blend can be further subjected to granulation or extrusion and the granulate or extrudate can be formed into a tablet or filled into a capsule, using routine techniques known in the art. Such matrix dosage forms can contain the active agent in the amount of from about 10 to about 85% on a weight-to-weight basis based on the final weight of the dosage form. The remainder of the dosage can contain the above-described ingredients in amounts that can be adjusted to achieve the desired active agent release profile, techniques of which are known in the art.

The present invention also contemplates that the matrix dosage forms described herein, can, such as after being filled into capsules or compressed into tablets, be subsequently coated with one or more enteric coatings. The enteric coatings that can be used for such coatings are described in more detail herein. For example, such dosage forms can be prepared by mixing at least one active agent and at least one pharmaceutically acceptable excipient to obtain a powder blend. The powder blend can then be enteric coated, can be compressed into a tablet that can be enteric coated or can be filled into a capsule which can be enteric coated. Also, the powder blend can be subjected to further granulation using routine techniques known in the art and the resulting granules enteric coated. The resulting granules can then be filled into a capsule and the capsule coated with at least one enteric coating, using routine techniques known in the art.

Another system that can be used to make the dosage forms of the present invention is a reservoir system. In this system, at least one core containing or comprising at least one active agent is coated or layered with at least one pharmaceutically acceptable coating, layer or membrane. The coating, layer or membrane, and its relative quantity, offers a predetermined resistance to active agent diffusion from the reservoir to the gastrointestinal tract. Thus, the active agent is gradually released from the core into the gastrointestinal tract, thereby providing a desired sustained release of the at least one active agent.

As mentioned briefly above, reservoir systems and methods for making such dosage forms are well known in the art. For example, U.S. Patent Nos. 5,286,497 and 5,737,320, describe such dosage forms and their methods of production. In the dosage forms of the present invention, the core(s) can be a granule, bead, pellet, particulate, microsphere, mini tablet, tablet or agglomerate. The core can be made in a variety of different ways. For example, the core can comprise a mixture of at least one active agent and at least one of the rate-controlling mechanisms described previously herein and, optionally, at least one of the pharmaceutically acceptable excipients described previously herein. Alternatively, the core can comprise at least one active agent and, optionally, at least one pharmaceutically acceptable excipient and can be further surrounded or coated with at least one rate-controlling mechanism. Alternatively, the core can comprise an inert substrate onto which is applied at least one active agent and, optionally, at least one pharmaceutically acceptable excipient. In addition, the substrate can be further surrounded or coated with at least one rate-controlling mechanism, at least one non-rate-controlling layer, at least one enteric coating or any combinations thereof.

Optionally, the core may also contain one or more non-rate-controlling layers as described previously herein. The location of the non-rate-controlling layer in the formulation is not critical. For example, the non-rate-controlling layer may be present between the core and an enteric coating or other polymeric coating. Alternatively, the non-rate-controlling layer may surround or coat an enteric coating or other polymeric coating.

The core can contain the active agent in the amount of from about 10 to about 99% on a weight-to-weight basis based on the final weight of the core. The reminder of the core can contain the above-described ingredients in amounts that can be adjusted to achieve the desired active agent release profile, techniques of which are known in the art.

The core can be produced by using routine techniques known in the art such as, but not limited to, direct blending, dry granulation (roller compaction), wet granulation (high shear granulation), milling or sieving, drying (if wet granulation is used), extrusion/spheronization, balling or compression, and, optionally, coating.

The second major component of a reservoir system is at least one coating, layer or membrane for use in controlling the release of the active agent from the dosage form. An example of a coating, layer or membrane that can be used is a polymeric coating. Examples of suitable polymers that can be used include, but are not limited to, ethylcellulose, cellulose acetate, cellulose propionate (lower, medium or higher molecular weight), cellulose acetate propionate, cellulose acetate butyrate, cellulose acetate phthalate, cellulose triacetate, poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), poly(ethylene), poly(ethylene) low density, poly(ethylene) high density, poly(propylene), poly(ethylene oxide), poly(ethylene terephthalate), poly(vinyl isobutyl ether), poly(vinyl acetate), poly(vinyl chloride) or polyurethane or mixtures thereof.

The polymeric coating may be applied to the core using methods and techniques known in the art. Examples of suitable coating devices include fluid bed coaters and pan coaters. The application techniques are described in more detail in: i) Aqueous polymeric coatings for pharmaceutical compositions, ed. J. W. McGinity, Marcel Dekker, Inc. New York, New York (1997); and ii) Pharmaceutical compositions: Tablets Vol. 3. ed. H. A. Lieberman, L. Lachman and J. B. Schwartz, Marcel Dekker, Inc. New York, New York pp. 77-287, (1990).

Another coating, layer or membrane that can be applied to the core is at least one enteric coating. One or more enteric coatings can be applied on to the core (the core may or may not contain one or more rate-controlling layers, non-rate-controlling layers or combinations of rate-controlling layers and non-rate controlling layers). For example, an enteric coating may be dispersed or dissolved in either water or in a suitable organic solvent and then sprayed on to the core or applied as a dry coating on to the core. Any enteric coating can be used in the present invention, including, but not limited to, solutions or dispersions of methacrylic acid and methacrylic ester copolymers, cellulose acetate phthalate, hydroxypropyl methylcellulose phthalate, hydroxypropyl methylcellulose acetate succinate, polyvinyl acetate phthalate, ethyl acrylate/methacrylic acid copolymers, cellulose acetate trimellitate, shellac and combinations thereof. For environmental reasons, aqueous based coatings can be used in the present invention as well. Examples of aqueous based coatings that can be used include, but are not limited to, methacrylic acid and methacrylic ester copolymers, hydroxypropyl methylcellulose acetate succinate, ethyl acrylate/methacrylic acid copolymers, cellulose acetate phthalate and combinations thereof.

The enteric coating can be formed as a single or multiple layers. The thickness of the coating can be readily determined by those skilled in the art, but must be sufficient to protect the dosage form in the acidic environment of the stomach.

The enteric coating(s) may contain one or more pharmaceutically acceptable plasticizers (in order to obtain desirable mechanical properties, such as, but not limited to, improved flexibility and strength of the enteric coating), such as, but not limited to, triacetin, citric acid esters, phthalic acid esters, dibutyl sebacate, cetyl alcohol, polyethylene glycols, polysorbates. The type and amount of plasticizer used will depend upon the intended composition of the enteric coating and can be readily determined by one skilled in the art. In addition to one or more plasticizers, the enteric coating can also contain anti-caking agents such as talc, as well as disperants, colorants, pigments, anti-foaming agents as well as other pharmaceutically acceptable agents to increase the thickness of the enteric coating and/or to regulate or modulate the diffusion of acidic gastric juices into the core.

If one or more enteric coatings are used, a coating between the core and the enteric coating can also be used (such a coating is frequently referred to as a "subcoating"). Any film forming polymer can be used as a subcoating. For example, polymers such as polyvinyl alcohol, hydroxypropyl cellulose, hydroxypropyl methyl cellulose can be used.

In an osmotic pump system, a core is encased by a semipermeable membrane having at least one orifice. The semipermeable membrane is permeable to water, but impermeable to the active agent. When the system is exposed to body fluids, water will penetrate through the semipermeable membrane into the tablet core containing osmotic excipients and at least one active agent. Osmotic pressure increases within the dosage form and the active agent is released through the orifice in an attempt to equalize pressure.

In more complex pumps, the core can contain multiple internal compartments. For example, the first compartment may contain at least one active agent and the second compartment may contain at least one polymer that swells on contact with fluid. After ingestion, the polymer swells into the active agent containing compartment at a predetermined rate and forces the active agent from the dosage form at that rate.

Pulsed release systems are also well known to those skilled in the art. Pulsed release systems release at least one active agent in pulses (namely, at different time points). Pulsed release systems also may include a combination of immediate release and extended release. Multiple configurations are suitable for pulsed release dosage forms of the active agent.

Osmotic pumps are well known in the art and have been described in the literature. For example, U. S. Patent Nos. 4,088,864, 4,200,098, and 5,573,776; describe osmotic pumps and methods for their manufacture.

Generally, osmotic pumps are typically formed by compressing a tablet of an osmotically active drug (or an osmotically inactive drug in combination with an osmotically active agent or osmagent) and then coating the tablet with a semipermeable membrane that is permeable to an exterior aqueous-based fluid but impermeable to the passage of drug and/or osmagent. One or more delivery orifices may be drilled through the semipermeable membrane wall. Alternatively, orifice(s) through the wall may be formed *in situ* by incorporating leachable pore forming materials in the wall. In operation, the exterior aqueous based fluid is imbibed through the semipermeable membrane wall and contacts the at least one active agent to form a solution or suspension of the active agent. The active agent solution or suspension is then "pumped" out through the orifice as fresh fluid is imbibed through the semipermeable membrane.

As mentioned previously herein, osmotic pumps may contain multiple distinct compartments. The first compartment may contain the active agent as described above, and the second compartment may contain an expandable driving member consisting of a layer of a swellable hydrophilic polymer, which operates to diminish the volume occupied by the active agent, thereby delivering the active agent from the device at a controlled rate over an extended period of time. Alternatively, the compartments may contain separate doses of at least one active agent

Semipermeable membranes that can be used include, but are not limited to, semipermeable polymers known in the art as osmosis and reverse osmosis membranes, such as cellulose acylate, cellulose diacylate, cellulose triacylate, cellulose acetate, cellulose diacetate, cellulose triacetate, agar acetate, amylose triacetate, beta glucan acetate, acetaldehyde dimethyl acetate, cellulose acetate ethyl carbamate, polyamides, polyurethanes, sulfonated polystyrenes, cellulose acetate phthalate, cellulose acetate methyl carbamate, cellulose acetate succinate, cellulose acetate dimethyl aminoacetate, cellulose acetate ethyl carbamate, cellulose acetate chloracetate, cellulose dipalmitate, cellulose dioctanoate, cellulose dicaprylate, cellulose dipentanlate, cellulose acetate valerate, cellulose acetate succinate, cellulose propionate succinate, methyl cellulose, cellulose acetate p-toluene sulfonate, cellulose acetate butyrate, cross-linked selectively semipermeable polymers formed by the coprecipitation of a polyanion and a polycation as disclosed in U. S. Patent Nos. 3,173,876, 3,276,586, 3,541,005, 3,541,006, and 3,546,142, semipermeable polymers as disclosed by Loeb and Sourirajan in U. S. Patent No. 3,133,132, lightly cross-linked polystyrene derivatives, cross-linked poly(sodium styrene sulfonate), poly(vinylbenzyltrimethyl ammonium chloride), cellulose acetate having a degree of substitution up to 1 and an acetyl content up to 50%, cellulose diacetate having a degree of substitution of 1 to 2 and an acetyl content of 21 to 35%, cellulose triacetate having a degree of substitution of 2 to 3 and an acetyl content of 35 to 44.8%, as disclosed in U. S. Patent No. 4,160,020.

The osmotic agent present in the pump, which may be used when the at least one active agent itself is not sufficiently osmotically active, are osmotically effective compounds soluble in the fluid that enters the pump, and exhibits an osmotic pressure gradient across the semipermeable wall against the exterior fluid. Osmotically effective osmagents useful for the present purpose include magnesium sulfate, calcium sulfate, magnesium chloride, sodium chloride, lithium chloride, potassium sulfate, sodium carbonate, sodium sulfite, lithium sulfate, potassium chloride, sodium sulfate, d-mannitol, urea, sorbitol, inositol, raffinose, sucrose, glucose, hydrophilic polymers such as cellulose polymers, mixtures thereof, and the like. The osmagent can be present in an excess amount, and it can be in any physical form, such as particle, powder, granule, and the like. The osmotic pressure in atmospheres of osmagents suitable for the invention will be greater than zero and generally up to about 500 atm, or higher.

The expandable driving member can be a swellable, hydrophilic polymer which interacts with water and aqueous biological fluids and swells or expands to an equilibrium state. The polymers exhibit the ability to swell in water and retain a significant portion of the imbibed water within the polymer structure. The polymers swell or expand to a very high degree, usually exhibiting a 2 to 50 fold volume increase. The polymers can be cross-linked or may not be cross-linked. The swellable, hydrophilic polymers can be lightly cross-linked, such cross-links being formed by covalent ionic bonds or hydrogen bonds. The polymers can be of plant, animal or synthetic origin. Hydrophilic polymers that can be used in the present invention include poly(hydroxy alkyl methacrylate) having a molecular weight from 30,000 to 5,000,000; kappa carrageenan, polyvinylpyrrolidone having molecular weight of from 10,000 to 360,000; anionic and cationic hydrogels; polyelectrolyte complexes; poly(vinyl alcohol) having a low acetate residual, cross-linked with glyoxal, formaldehyde, or glutaraldehyde and having a degree of polymerization from 200 to 30,000; a mixture of methyl cellulose; cross-linked agar and carboxymethyl cellulose; a water insoluble, water swellable copolymer produced by forming a dispersion of finely divided copolymer of maleic anhydride with styrene, ethylene, propylene, butylene or isobutylene cross-linked with from 0.001 to about 0.5 moles of saturated cross-linking agent per mole of maleic anhydride in copolymer; water swellable polymers ofN-vinyl lactams, and the like.

The term "orifice" as used herein refers to means and methods suitable for releasing the at least one active agent from an osmotic system. The expression includes one or more apertures or orifices which have been bored through the semipermeable membrane by mechanical procedures. Alternatively, the orifice can be formed by incorporating an erodible element, such as a gelatin plug, in the semipermeable membrane. In cases where the semipermeable membrane is sufficiently permeable to the passage of active agent, the pores in the membrane may be sufficient to release the at least one active agent in amounts sufficient to meet the plasma threshold. In such cases, the term "passageway" refers to the pores within the membrane wall even though no bore or other orifice has been drilled there through. A detailed description of osmotic passageways and the maximum and minimum dimensions for a passageway are disclosed in United States Patent Nos. 3,845,770 and 3,916,899,

Osmotic pumps can be made using routine techniques known to those skilled in the art. For example, in one embodiment, the at least one active agent, at least one rate-controlling mechanism and optionally at least one pharmaceutically acceptable excipient may be housed in one area of the compartment adjacent to the passageway, are pressed into a solid having a dimension that corresponds to the internal dimensions of the area of the compartment the at least one active agent will occupy, or the active agent, rate-controlling mechanism and excipients and a solvent are mixed into a solid or semisolid form by conventional methods such as, but not limited to, ball milling, calendaring, stirring or roll milling, and then pressed into a preselected shape. Next, a layer of a hydrophilic polymer is placed in contact with the layer of drug in a like manner, and the two layers surrounded with a semipermeable wall. The layering of drug formulation and hydrophilic polymer can be fabricated by conventional two-layer press techniques. The wall can be applied by molding, spraying or dipping the pressed shapes into a wall forming material. Another technique that can be used for applying the wall is the air suspension procedure. This procedure consists of suspending and tumbling the pressed agent and dry hydrophilic polymer in a current of air and a wall forming composition until the wall is applied to the agent-hydrophilic polymer composite. The air suspension procedure is described in U. S. Patent No. 2,799,241; J. Am. Pharm. Assoc., 48:451-459 (1979). Other manufacturing procedures are described in Modern Plastics Encyclopedia, Vol. 46, pp. 62-70 (1969); and in Pharmaceutical Sciences, by Remington, Fourteenth Edition, pp. 1626-1678 (1970), published by Mack Publishing Company, Easton, PA.

As mentioned previously herein, the present invention encompasses any dosage forms suitable for oral administration including, but are not limited to, capsules, tablets, pills, powders, etc. Liquid dosage forms for oral administration are also contemplated herein and include, but are not limited to, pharmaceutically acceptable emulsions, solutions, suspensions or syrups.

One of ordinary skill in the art will appreciate that effective amount of at least one active agent contained in any dosage form can be determined empirically using routine experimentation. Actual dosage levels of active agent in the formulations of the present invention may be varied to obtain an amount of active agent that is effective to obtain a desired therapeutic response. The selected dosage level depends upon the desired therapeutic effect, the route of administration, the potency of the active agent administered, the desired duration of treatment and other factors.

IV. Other Pharmaceutically Acceptable Active Agents and Combination Therapy

The solid dosage forms of the present invention can also comprise pharmaceutically acceptable active agents other than 2-[4-(4-chlorobenzoyl)phenoxy]-2-methyl-propanoic acid, salts of 2-[4-(4-chlorobenzoyl)phenoxy]-2-methyl-propanoic acid and/or buffered 2-[4-(4-chlorobenzoyl)phenoxy]-2-methyl-propanoic acid (said active agents other than 2-[4-(4-chlorobenzoyl)phenoxy]-2-methyl-propanoic acid, salts of 2-[4-(4-chlorobenzoyl)phenoxy]-2-methyl-propanoic acid and/or buffered 2-[4-(4-chlorobenzoyl)phenoxy]-2-methyl-propanoic acid are collectively referred to herein as "other agents"). Additionally, these other agents can be co-packaged with the dosage forms of the present invention or co-administered as one or more separate and distinct dosage forms along with the solid dosage forms of the present invention. These other agents can be from the same therapeutic class as the active agent (e.g., lipid-regulating pharmaceutical agents) or can be from different therapeutic classes (e.g., anti-hypertensive pharmaceutical agents). Examples of other agents that can be included in or as part of the dosage forms of the present invention or co-administered with the formulations of the present invention include, but are not limited to, lipid-regulating agents, anti-hypertensive agents, anti-diabetic agents, weight-loss agents, antiretroviral agents, anti-platelet agents or vitamins and minerals.

Examples of such other active agents, include, but are not limited to:

- **lipid regulating agents** (such as, but not limited to, atorvastatin, simvastatin, fluvastatin, pravastatin, lovastatin, cerivastatin, rosuvastatin, pitavastatin, clofibric acid, niacin/nicotinic acid, torcetrapib, colestipol, omega-3 acid ethyl esters, colesevelam, cholestyramine, ezetimibe, MD-0727, gemfibrozil or probucol);
- **anti-hypertensive agents** (such as, but not limited to, amlodipine, benazepril, benidipine, candesartan, captopril, carvedilol, darodipine, dilitazem, diazoxide, doxazosin, enalapril, epleronone, eprosartan, felodipine, fenoldopam, fosinopril, guanabenz, iloprost, irbesartan, isradipine, lercardinipine, lisinopril, losartan, minoxidil, nebivolol, nicardipine, nifedipine, nimodipine, nisoldipine, omapatrilat, phenoxybenzamine, prazosin, quinapril, reserpine, semotiadil, sitaxsentan, terazosin, telmisartan, labetolol, valsartan, triamterene, metoprolol, methyldopa, ramipril, olmesartan, timolol, verapamil, clonidine, nadolol, bendromethiazide, torsemide, hydrochlorothiazide, spinronolactone, perindopril, hydralazine, betaxolol, furosimide, penbutolol, acebutolol, atenolol, bisoprolol, nadolol, penbutolol, pindolol, propranolol, timolol, indapamide, trandolopril, amiloride, moexipril, metolozone, or valsartan);
- **anti-diabetic agents** (such as, but not limited to, acarbose, oral insulin, acetohexamide, chlorpropamide, ciglitazone, farglitazar, glibenclamide, gliclazide, glipizide, glucagon, glyburide, glymepiride, miglitol, pioglitazone, nateglinide, pimagedine, repaglinide, rosiglitazone, tolazamide, tolbutamide, triampterine or troglitazone);
- **weight-loss agents** (such as, but not limited to, phentermine, phendimetrazine, benzphetamine, diethylpropion, sibutramine, orlistat or rimonabant);
- **antiretroviral agents** (such as but not limited to, amprenavir, tiprinavir, lamivudine, indinavir, emtricitabine, abacavir, enfuvirtide, saquinavir, lopinavir, ritonavir, fosamprenavir, delaviradine mesylate, zidovudine, atazanavir, efavirenz, tenofivir, emtricitabine, didanosine, nelfinavir, nevirapine, or stavudine);
- **anti-platelet agents** (such as, but not limited to, aspirin, cilostazol, or pentoxifylline); or
- **vitamins, minerals or combinations of vitamins and minerals** (such as, but not limited to folic acid, calcium, or iron).

As alluded to briefly herein, the other agent(s) can be included in the dosage forms of the present invention. The precise location and amount of the other agent(s) to be included in the dosage forms of the present invention can be readily determined by one skilled in the art and will depend upon the other agent(s) to be delivered as well as the treatment to be provided. When the other agent(s) is included in the dosage forms of the present invention, then the active agent (at least one of 2-[4-(4-chlorobenzoyl)phenoxy]-2-methyl-propanoic acid, salts of 2-[4-(4-chlorobenzoyl)phenoxy]-2-methyl-propanoic acid and/or buffered 2-[4-(4-chlorobenzoyl)phenoxy]-2-methyl-propanoic acid) and other agent(s) are administered in a single formulation. Alternatively, the other agent(s) and the active agent can each be contained in separate dosage forms and then administered simultaneously or successively.

V. Methods of Using the Dosage Forms of the Present Invention

The oral formulations of the present invention can be used in treating a variety of conditions. When the oral formulations of the present invention contain at least one active agent and combinations thereof, such formulations can be used in treating conditions such as hypercholesterolemia, hypertriglyceridemia, cardiovascular disorders, coronary heart disease, peripheral vascular disease (including symptomatic carotid artery disease) and metabolic disorders (such as, but not limited to, obesity, diabetes, hyperphagia, endocrine abnormalities, triglyceride storage disease, Bardet-Biedl syndrome, Lawrence-Moon syndrome, Prader-Labhart-Willi syndrome, hypophagia, anorexia and cachexia). Moreover, these formulations can be used as adjunctive therapy for the reduction of low density lipoprotein cholesterol (hereinafter "LDL-C"), total cholesterol (hereinafter "total-C"), triglycerides, and apolipoprotein B (hereinafter "Apo B") in adult subjects with primary hypercholesterolemia or mixed dyslipidemia (Fredrickson Types IIa and IIb). These compositions can also be used as adjunctive therapy for treatment of adult subjects with hypertriglyceridemia (Fredrickson Types IV and V hyperlipidemia). Markedly elevated levels of serum tryglycerides (for example, >2000 mg/dL) may increase the risk of developing pancreatitis. Additionally, these formulations can further be used for other indications where lipid-regulating agents are typically used.

By way of example, and not of limitation, examples of the present invention will now be given.

**Formulations 5, 6, 9, 10, 11, 12 and 13 hereinunder are formulations according to the present invention, the other Formulations not containing the components of appended claim 1.**

EXAMPLE 1: Pharmaceutical Formulations

The following formulations were prepared and tested *in vitro* and as well as in healthy human subjects: (a) three mini-tablet formulations (each having no enteric coating) containing 2-[4-(4-chlorobenzoyl)phenoxy]-2-methyl-propanoic acid (Formulations 1, 2, and 8); (b) six (6) enteric coated mini-tablet formulations containing choline salt of 2-[4-(4-chlorobenzoyl)phenoxy]-2-methyl-propanoic acid (Formulation 5 and 9-13); (c) single-unit enteric coated tablet containing choline salt of 2-[4-(4-chlorobenzoyl)phenoxy]-2-methyl- propanoic acid (Formulation 6); and (d) coated granules containing 2-[4-(4-chlorobenzoyl)phenoxy]-2-methyl-propanoic acid in capsules (Formulation 7). Formulations 3 and 4 (containing a choline salt of 2-[4-(4-chlorobenzoyl)phenoxy]-2-methyl-propanoic acid) were prepared, tested *in vitro,* but not tested in human subjects. In addition, a prototype osmotic pump containing choline salt of 2-[4-(4-chlorobenzoyl)phenoxy]-2-methyl-propanoic acid was tested *in vitro,* but was not tested in humans.

The compositions of the above formulations are listed below in Tables 15 to 24 (these formulations are referred to in subsequent examples as "Formulation 1" or "Form 1", "Formulation "5" or "Form 5", or "Formulation 10" or "Form 10", etc. respectively). All formulations are manufactured using a similar manufacturing process, except for the coating process. Specifically, a high shear granulation process is used to incorporate the active ingredient and pharmaceutically acceptable excipients into the granules. The granules are sieved, dried, sieved (or milled) and mixed with the remaining ingredients to form the final powder blend. From there, various processes take place. For all the formulations with the exception of Formulation 7, the final powder blend was further compressed using a rotary compression machine to produce tablets. For Formulations 5-6 and 9-13, the tablets are coated with an enteric coating. Formulations 1, 2, 3 and 8 were not coated. Powder blends from Formulation 7 and the mini-tablets of Formulation 4 were coated with a rate-controlling mechanism (namely, a rate-controlling polymer).

A detailed description of all of the manufacturing processes is provided below.

Table 15

| Composition of Formulation 1 (mini-tablets without enteric coating in capsules). The size of each tablet is approximately 2 mm in diameter. The dose strength of each capsule is 130 mg free acid equivalent. ||
|---|---|
| Ingredient | % W/W (based on core tablet weight) |
| Core | |
| Intra-granular ||
| 2-[4-(4-chlorobenzoyl)phenoxy]-2-methyl-propanoic acid | 35% |
| HPMC K15M | 10% |
| Avicel PH101 | 12% |
| PVP 30 | 2.25% |
| Extra-granular ||
| Colloidal silicon dioxide | 0.5% |
| HPMC K15M | 40% |

(continued)

| Extra-granular | |
|---|---|
| Magnesium stearate | 0.25% |
| Coating | Not applied |

**Manufacturing Process for Formulation 1**

| Step | |
|---|---|
| 1 | Weight the appropriate amount for each ingredient. Charge intra-granular ingredients into a high shear granulator and dry mix for approximately 1 minute. |
| 2 | Add appropriate amount of water into the above high shear granulator and start granulation. |
| 3 | Stop water addition and continue granulation for an appropriate amount of time until a pre-determined end-point (for example, power or time). |
| 4 | Discharge the above granulation through a mesh screen and place in a fluid bed dryer for drying. |
| 5 | Sieve the dry granulation. |
| 6 | Screen extra-granular HPMC K15M and colloidal silicon dioxide through a mesh screen, charge into a blender and blend for 2 minutes. |
| 7 | Charge dry and sieved granulation from step 5 into the blender and blend for 5 minutes. |
| 8 | Screen magnesium stearate through a mesh screen, charge into the above blender and blend for 1 minute. |
| 9 | Discharge the above powder blend from step 8 into a rotary press machine. Compress tablets into the target tablet weight. |
| 10 | Encapsulate coated tablets into capsules. Conduct metal detection of capsules and discard rejected capsules. Package acceptable capsules into bottles for clinical supplies. |

Table 16

| Composition of Formulation 2 (mini-tablets without enteric coating in capsules). The size of each tablet is approximately 3.2 mm in diameter. The dose strength of each capsule is 130 mg free acid equivalent. | |
|---|---|
| Ingredient | % W/W (based on core tablet weight) |
| Core | |
| 2-[4-(4-chlorobenzoyl)phenoxy]-2-methyl-propanoic acid | 40% |
| Dibasic calcium phosphate | 15% |
| Avicel PH101 | 24% |
| PVP 30 | 5% |
| Lactose monohydrate | 15% |
| Magnesium stearate | 1% |
| Coating | Not applied |

**Manufacturing Process for Formulation 2**

| Step | |
|---|---|
| 1 | Weight the appropriate amount for each ingredient. Charge all ingredients (except magnesium stearate) into a high shear granulator and dry mix for approximately 1 minute. |
| 2 | Add appropriate amount of water into the above high shear granulator and start granulation. |
| 3 | Stop water addition and continue granulation for an appropriate amount of time a pre-determined end-point (for example, power or time). |
| 4 | Discharge the above granulation through a mesh screen and place in an over for drying. |
| 5 | Mill the dry granulation and discharge into blender. |
| 6 | Screen magnesium stearate through a mesh screen, charge into the above blender and blend for an appropriate amount of time. |
| 7 | Discharge the above powder blend into a rotary press machine. Compress tablets into the target tablet weight. |
| 8 | Manually encapsulate coated tablets into capsules. Conduct metal detection of capsules and discard rejected capsules. Package acceptable capsules into bottles for clinical supplies. |

Table 17

| Composition of Formulation 3 (single unit HPMC-based tablets without coating). The dose strength of each tablet is 130 mg free acid equivalent. | |
|---|---|
| Ingredient | % W/W (based on core tablet weight) |
| Core | |
| Intra-granular | |
| 2-[4-(4-chlorobenzoyl)phenoxy]-2-methyl-propanoic acid Choline salt | 61.0% |
| HPMC K15M | 30.25% |
| Avicel PH101 | 5.0% |
| PVP 30 | 3.0% |
| Extra-granular | |
| Colloidal silicon dioxide | 0.25% |
| Magnesium stearate | 0.5% |
| Coating | |
| None | |

**Manufacturing Process for Formulation 3**

| Step | |
|---|---|
| 1 | Weigh the appropriate amount for each ingredient. Charge intra-granular ingredients into a high shear granulator and dry mix until uniform. |
| 2 | Add appropriate amount of water into the above high shear granulator and start granulation. |
| 3 | Stop water addition and continue granulation for an appropriate amount of time until a pre-determined end-point (for example, power or time). |
| 4 | Discharge the above granulation through a mesh screen and place in an over for drying. |
| 5 | Pass the dry granulation through a mesh screen. Mill granulation retained on the screen. |

(continued)

| Step | |
|---|---|
| 6 | Charge screened and milled granulations into a V-blender. Screen colloidal silicon dioxide through a mesh screen and charge into the same V-blender. Blend until uniform. |
| 7 | Screen magnesium stearate through a mesh screen and charge into the above V-blender. Blend until uniform. |
| 8 | Discharge the above powder blend into a rotary press machine. Compress tablets into the target tablet weight. |
| 9 | Package tablets into bottles for clinical supplies. |

Table 18

| Composition of Formulation 4 (coated mini-tablets in capsule). The dose strength of each capsule is 130 mg free acid equivalent. | |
|---|---|
| Ingredient | % W/W (based on core tablet weight) |
| Core | |
| Intra-granular | |
| 2-[4-(4-chlorobenzoyl)phenoxy]-2-methyl-propanoic acid Choline salt | 40.0% |
| Avicel PH101 | 24.0% |
| Dicalcium phosphate | 15.0% |
| Lactose monohydrate | 15.0% |
| PVP 30 | 5.0% |
| Extra-granular | |
| Magnesium stearate | 1.0% |
| Coating | |
| Surelease E-7-19010 (solid content) | 6.0% |
| Opadry Clear | 1.5% |

**Manufacturing Process for Formulation 4**

| Step | |
|---|---|
| 1 | Weigh the appropriate amount for each ingredient. Charge intragranular ingredients into a high shear granulator and dry mix until uniform. |
| 2 | Add appropriate amount of water into the above high shear granulator and start granulation. |
| 3 | Stop water addition and continue granulation for an appropriate amount of time until a pre-determined end-point (for example, power or time). |
| 4 | Discharge the above granulation through a mesh screen and place in an over for drying. |
| 5 | Pass the dry granulation through a mesh screen. Mill granulation retained on the screen. |
| 6 | Charge screened and milled granulations into a V-blender. |
| 7 | Screen magnesium stearate through a mesh screen and charge into the above V-blender. Blend until uniform. |
| 8 | Discharge the above powder blend into a rotary press machine. Compress tablets into the target tablet weight. |
| 9 | Charge water, talc, and triethyl citrate into a container and mix, and then mix well with the Surelease and Opadry. |

(continued)

| Step | |
|------|---|
| 10 | Charge above compressed tablets into a Wuster coater and start coating using the pre-mixed coating solution. Stop coating when the target coating level is achieved. |
| 11 | Manually encapsulate coated tablets into capsules. Conduct metal detection of capsules and discard rejected capsules. Package acceptable capsules into bottles for clinical supplies. |

Table 19

| Composition of Formulation 5 (enteric-coated mini-tablets in capsule). The size of each tablet is approximately 3.2 mm in diameter. The dose strength of each capsule is 130 mg free acid equivalent. | |
|---|---|
| Ingredient | % W/W (based on core tablet weight) |
| Core | |
| Intra-granular | |
| 2-[4-(4-chlorobenzoyl)phenoxy]-2-methyl-propanoic acid Choline salt | 49.9% |
| HPMC K15M | 35.0% |
| Avicel PH101 | 11.1% |
| PVP 30 | 3% |
| Extra-granular | |
| Colloidal silicon dioxide | 0.5% |
| Magnesium stearate | 0.5% |
| Coating | |
| Eudragit® L30 D55 | 10.4% |
| Talc | 5.2% |
| Triethyl Citrate | 1.6% |

**Manufacturing Process for Formulation 5**

| Step | |
|------|---|
| 1 | Weight the appropriate amount for each ingredient. Charge intragranular ingredients into a high shear granulator and dry mix for 1 minute. |
| 2 | Add appropriate amount of water into the above high shear granulator and start granulation. |
| 3 | Stop water addition and continue granulation for an appropriate amount of time pre-determined end-point (for example, power or time). |
| 4 | Discharge the above granulation through a mesh screen and place in an over for drying. |
| 5 | Pass the dry granulation through a mesh screen. Mill granulation retained on the screen. |
| 6 | Charge screened and milled granulations into a V-blender. Screen colloidal silicon dioxide through a mesh screen and charge into the same V-blender. Blend for 5 minutes. |
| 7 | Screen magnesium stearate through a mesh screen and charge into the above V-blender. Blend for 2 minutes. |
| 8 | Discharge the above powder blend into a rotary press machine. Compress tablets into the target tablet weight. |

(continued)

| Step | |
|------|---|
| 9 | Charge water, talc, and triethyl citrate into a container and mix, and then mix well with the Eudragit® L30 D55 suspension. |
| 10 | Charge above compressed tablets into a Wurster coater and start coating using the pre-mixed coating solution. Stop coating when the target coating level is achieved. |
| 11 | Manually encapsulate coated tablets into capsules. Conduct metal detection of capsules and discard rejected capsules. Package acceptable capsules into bottles for clinical supplies. |

Table 20

| Composition of Formulation 6 (single unit enteric-coated tablet). The dose strength of each tablet is 130 mg free acid equivalent. | |
|---|---|
| Ingredient | % W/W (based on core tablet weight) |
| Core | |
| Intra-granular | |
| 2-[4-(4-chlorobenzoyl)phenoxy]-2-methyl-propanoic acid Choline salt | 65% |
| HPMC K15M | 15% |
| Avicel PH101 | 10.75% |
| PVP 30 | 3% |
| Extra-granular | |
| Avicel PH102 | 5.0% |
| Colloidal silicon dioxide | 0.75% |
| Magnesium stearate | 0.5% |
| Coating | |
| Eudragit® L30 D55 | 9.5% |
| Talc | 4.8% |
| Triethyl Citrate | 1.4% |

**Manufacturing Process for Formulation 6**

| Step | |
|------|---|
| 1 | Weigh the appropriate amount for each ingredient. Charge intragranular ingredients into a high shear granulator and dry mix until uniform. |
| 2 | Add appropriate amount of water into the above high shear granulator and start granulation. |
| 3 | Stop water addition and continue granulation for an appropriate amount of time until a pre-determined end-point (for example, power or time). |
| 4 | Discharge the above granulation through a mesh screen and place in an over for drying. |
| 5 | Pass the dry granulation through a mesh screen. Mill granulation retained on the screen. |
| 6 | Charge screened and milled granulations into a V-blender. Screen colloidal silicon dioxide and Avicel® through a mesh screen and charge into the same V-blender. Blend until uniform. |

(continued)

| Step | |
|---|---|
| 7 | Screen magnesium stearate through a mesh screen and charge into the above V-blender. Blend until uniform. |
| 8 | Discharge the above powder blend into a rotary press machine. Compress tablets into the target tablet weight. |
| 9 | Charge water, talc, and triethyl citrate into a container and mix, and then mix well with the Eudragit® L30 D55 suspension. |
| 10 | Charge above compressed tablets into a coater and start coating using the pre-mixed coating solution. Stop coating when the target coating level is achieved. |
| 11 | Package acceptable tablets into bottles for clinical supplies. |

Table 21

| Composition of Formulation 7 (coated granules in capsules). The dose strength of each capsule is 130 mg free acid equivalent. | |
|---|---|
| Ingredient | % W/W (based on fill weight) |
| Intragranular | |
| 2-[4-(4-chlorobenzoyl)phenoxy]-2-methyl-propanoic acid | 91.4% |
| Eudragit@ NE 30D | 4.9% |
| PVP K30 | 2.7% |
| Extra-granular | |
| Magnesium stearate | 1.0% |
| Coating | |
| Eudragit® NE 30D | 7.0 |
| Talc | 7.0 |
| Coating | |
| Eudragit® NE 30D | 7.0 |
| Talc | 7.0 |

**Manufacturing Process for Formulation 7**

| Step | |
|---|---|
| 1 | Weigh the appropriate amount for each ingredient. Charge intragranular ingredients into a high shear granulator and dry mix until uniform. |
| 2 | Add appropriate amount of Eudragit® NE30D suspension into the above high shear granulator and start granulation. |
| 3 | Stop Eudragit® NE30D addition and continue granulation for an appropriate amount of until a pre-determined end-point (for example, power or time). |
| 4 | Discharge the above granulation through a mesh screen and place in a fluid bed dryer for drying. |
| 5 | Sieve the dry granulation. |
| 7 | Charge dry and sieved granulation from step 5 into the blender and blend until uniform. |
| 8 | Screen magnesium stearate through a mesh screen, charge into the above blender and blend until uniform. |
| 9 | Charge water, talc into a container and mix, and then mix well with the Eudragit® NE30D suspension. |

(continued)

| Step | |
|------|---|
| 10 | Charge above granules from step 8 into a coater and start coating using the pre-mixed coating solution. Stop coating when the target coating level is achieved. |
| 11 | Encapsulate coated granules into capsules. Conduct metal detection of capsules and discard rejected capsules. Package acceptable capsules into bottles for clinical supplies. |

Table 22

| Composition of Formulation 8 (mini-tablets without coating in capsule). The size of each tablet is approximately 2.0 mm in diameter. The dose strength of each capsule is 130 mg free acid equivalent. | |
|---|---|
| Ingredient | % W/W (based on core tablet weight) |
| Intra-granular | |
| 2-[4-(4-chlorobenzoyl)phenoxy]-2-methyl-propanoic acid | 91.8% |
| Eudragit® NE 30D | 5.0% |
| PVP K30 | 2.8% |
| Extra-granular | |
| Magnesium stearate | 0.5% |

**Manufacturing Process for Formulations 8**

| Step | |
|------|---|
| 1 | Weigh the appropriate amount for each ingredient. Charge intra-granular ingredients into a high shear granulator and dry mix until uniform. |
| 2 | Add appropriate Eudragit® NE30D into the above high shear granulator and start granulation. |
| 3 | Stop Eudragit® NE30D addition and continue granulation for an appropriate amount of time until a pre-determined end-point (for example, power or time). |
| 4 | Discharge the above granulation through a mesh screen and place in a fluid bed dryer for drying. |
| 5 | Sieve the dry granulation. |
| 6 | Charge dry and sieved granulation from step 5 into the blender. |
| 7 | Screen magnesium stearate through a mesh screen, charge into the above blender and blend until uniform. |
| 8 | Discharge the above powder blend into a rotary press machine. Compress tablets into the target tablet weight. |
| 9 | Manually encapsulate tablets into capsules. Conduct metal detection of capsules and discard rejected capsules. Package acceptable capsules into bottles. |

Table 23

| Composition of Formulations 9, 10, and 11 (enteric-coated mini-tablets in capsules). The size of each tablet is approximately 3 mm in diameter. The dose strength of each capsule is 135 mg free acid equivalent. | | | |
|---|---|---|---|
| **Composition of Formulations 9,10 and 11** | | | |
| Ingredients | Formulation 9 (% w/w) | Formulation 10 (%w/w) | Formulation 11 (% w/w) |
| Intra-granular | | | |
| Fenofibric acid, choline salt | 57.5 | 65.5 | 71.5 |
| HPMC K15M | 37 | 27 | 21 |
| PVP K30 | 3 | 3 | 3 |
| Extra-granular | | | |
| HPC EXF | 3 | 3 | 3 |
| Colloidal Silicon Dioxide | 0.5 | 0.5 | 0.5 |
| Sodium stearyl fumarate | 1 | 1 | 1 |
| Coating | | | |
| Eudragit® L30-D55 | 9.9 | 9.9 | 9.9 |
| Talc | 4.55 | 4.55 | 4.55 |
| Triethyl citrate | 1.36 | 1.36 | 1.36 |

**Manufacturing Process for Formulations 9,10, and 11**

| Step | |
|---|---|
| 1 | Weigh the appropriate amount for each ingredient. Charge intragranular ingredients into a high shear granulator and dry mix until uniform. |
| 2 | Add appropriate amount of water into the above high shear granulator and start granulation. |
| 3 | Stop water addition and continue granulation for an appropriate amount of time until a pre-determined end-point (for example, power or time). |
| 4 | Discharge the above granulation through a mesh screen and place in an oven for drying. |
| 5 | Pass the dry granulation through a mesh screen. Mill granulation retained on the screen. |
| 6 | Charge screened and milled granulations into a V-blender. Screen colloidal silicon dioxide and HPC EXF through a mesh screen and charge into the same V-blender. Blend until uniform. |
| 7 | Screen magnesium stearate through a mesh screen and charge into the above V-blender. Blend until uniform. |
| 8 | Discharge the above powder blend into a rotary press machine. Compress tablets into the target tablet weight. |
| 9 | Charge water, talc, and triethyl citrate into a container and mix, and then mix well with the Eudragit® L30 D55 suspension. |
| 10 | Charge above compressed tablets into a Wurster coater and start coating using the pre-mixed coating solution. Stop coating when the target coating level is achieved. |
| 11 | Manually encapsulate coated tablets into capsules. Conduct metal detection of capsules and discard rejected capsules. Package acceptable capsules into bottles for clinical supplies. |

Table 24

| Composition of Formulations 12 and 13 (enteric-coated mini-tablets in capsules). The size of each tablet is approximately 3 and 4 mm, respectively. The dose strength of each capsule is 135 mg free acid equivalent. | | |
|---|---|---|
| **Composition of Formulations 12 and 13** | | |
| Ingredients | Formulation 12 (% w/w) | Formulation 13 (%w/w) |
| Intra-granular | | |
| Fenofibric acid, choline salt | 65.5 | 65.5 |
| HPMC K15M | 5 | 27 |
| HPMC K100LV | 22 | 0 |
| PVP K30 | 3 | 3 |
| Extra-granular | | |
| HPC EXF | 3 | 3 |
| Colloidal Silicon Dioxide | 0.5 | 0.5 |
| Sodium stearyl fumarate | 1 | 1 |
| Coating | | |
| Eudragit® L30-D55 | 9.9 | 9.9 |
| Talc | 4.55 | 4.55 |
| Triethyl citrate | 1.36 | 1.36 |

**Manufacturing Process for Formulations 12 and 13**

| step | |
|---|---|
| 1 | Weigh the appropriate amount for each ingredient. Charge intragranular ingredients into a high shear granulator and dry mix until uniform. |
| 2 | Add appropriate amount of water into the above high shear granulator and start granulation. |
| 3 | Stop water addition and continue granulation for an appropriate amount of time until a pre-determined end-point (for example, power or time). |
| 4 | Discharge the above granulation through a mesh screen and place in an oven for drying. |
| 5 | Pass the dry granulation through a mesh screen. Mill granulation retained on the screen. |
| 6 | Charge screened and milled granulations into a V-blender. Screen colloidal silicon dioxide and HPC EXF through a mesh screen and charge into the same V-blender. Blend until uniform. |
| 7 | Screen magnesium stearate through a mesh screen and charge into the above V-blender. Blend until uniform. |
| 8 | Discharge the above powder blend into a rotary press machine. Compress tablets into the target tablet weight. |
| 9 | Charge water, talc, and triethyl citrate into a container and mix, and then mix well with the Eudragit® L30 D55 suspension. |
| 10 | Charge above compressed tablets into a Wurster coater and start coating using the pre-mixed coating solution. Stop coating when the target coating level is achieved. |
| 11 | Manually encapsulate coated tablets into capsules. Conduct metal detection of capsules and discard rejected capsules. Package acceptable capsules into bottles for clinical supplies. |

**EXAMPLE 2: Dissolution Methods**

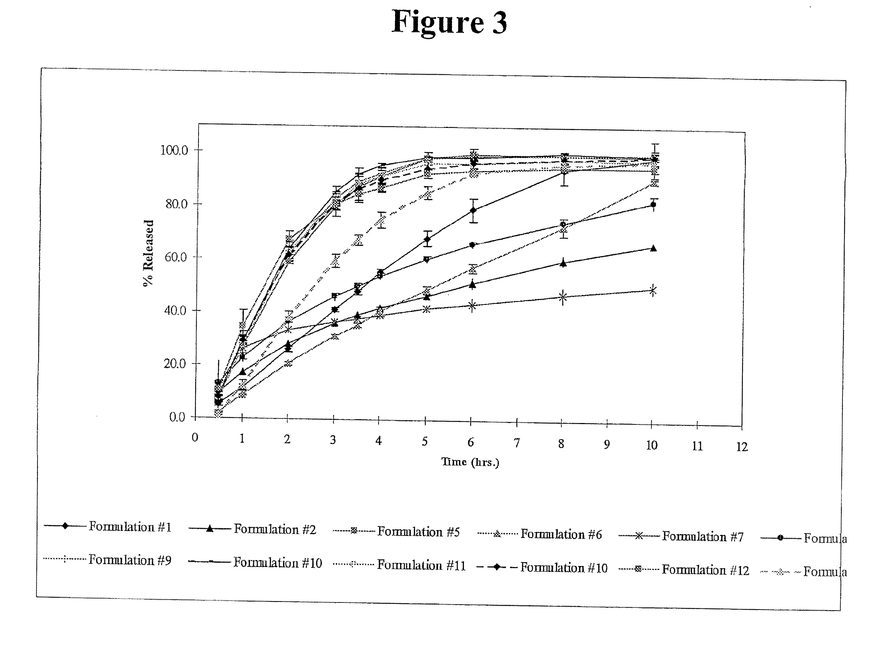

The dissolution data from Formulations 1-2 and 5-13 as described in Example 1 was collected and is depicted in Figure 3 using the single pH method.

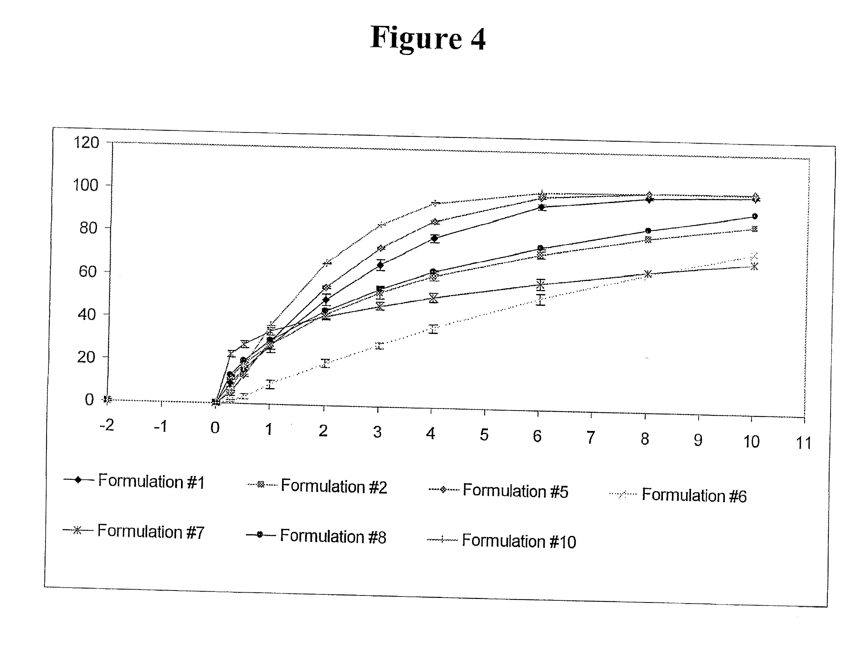

The dissolution data from Formulations 1-2 and 5-13 as described in Example 1 was collected and is depicted in Figure 4 using the dual pH method.

**Example 3: Human Biostudies**

The purpose of the studies described in Examples 4, 5, 6 and 7 was to determine the bioavailability of the Formulations 1-2 and 5-13. These utilized a Phase 1, single-dose, open-label study conducted according to a crossover design. The number of subjects varied from study to study. The number of subjects that entered the studies and completed at least a portion of the studies is noted in each of the examples described herein. Subjects entered the study and were assigned to receive one of the following regimens in each study period: (1) the Reference; (2) a test Formulation under high fat fed conditions; or (3) a test Formulation under fasting conditions. The sequences of regimens were such that a pre-determined number of subjects received all of the regimens upon completion of the study, while others received part of the regimes. A washout interval of typically about 14 days separated the dosing in two (2) consecutive periods. Adult male and female subjects in general good health were selected to participate in the study.

For a typical study, subjects were confined to the study site and supervised for approximately 6 days in each study period. Confinement in each period began in the afternoon on Study Day -1 (1 day prior to the dosing day) and ended after the collection of the 120-hour blood samples and scheduled study procedures were completed on the morning of Study Day 6. Strenuous activity during the confinement was not permitted.

With the exception of the breakfast on Study Day 1 in each period, subjects received a standard diet, providing approximately 34% calories from fat per day, for all meals during confinement. For those subjects receiving a test Formulation under fasting conditions, no food or beverage, except for water to quench thirst, was allowed beginning 10 hours before dosing and continuing until after the collection of the 4-hour blood sample on the following day (Study Day 1). No fluids were allowed for 1 hour before dosing and 1 hour after dosing. For breakfast on Study Day 1 in each period, subjects received a meal or no meal, corresponding to their assigned regimen. Subjects assigned to receive the test formulation under fed conditions received a high-fat breakfast that provided approximately1000 Kcal and 50% of calories from fat beginning 30 minutes prior to dosing.

On Study Day 1, all subjects were served lunch following collection of the 4-hour blood sample, dinner following collection of the 10-hour blood sample, and a snack approximately 4 hours after dinner. The meal content with the exception of breakfast was identical on the intensive pharmacokinetic sampling days (Study Day 1) of all four periods.

Typically, blood samples were collected from the subjects prior to dosing and at 0.5, 1, 2, 3, 4, 5, 6, 7, 8, 9, 10, 11, 12, 18, 24, 48, 72, 96 and 120 hours after dosing in each period. The blood samples were centrifuged to separate the plasma. The plasma samples were stored frozen until analyzed.

Plasma concentrations of fenofibric acid were determined using a validated liquid chromatographic method with mass spectrometric detection.

Values for the pharmacokinetic parameters of fenofibric acid were estimated using noncompartmental methods. First, the maximum observed plasma concentration ($C_{max}$) and the time to $C_{max}$ (peak time, $T_{max}$) were determined directly from the plasma concentration-time data. Second, the value of the terminal phase elimination rate constant ($\lambda_z$) was obtained from the slope of the least squares linear regression of the logarithms of the plasma concentration *versus* time data from the terminal log-linear phase of the profile. A minimum of three concentration-time data points was used to determine $\lambda_z$. The terminal phase elimination half-life ($t_{1/2}$) was calculated as $\ln(2)/\lambda_z$. Third, the area under the plasma concentration-time curve (AUC) from time 0 to time of the last measurable concentration ($AUC_t$) was calculated by the linear trapezoidal rule. The AUC was extrapolated to infinite time by dividing the last measurable plasma concentration ($C_t$) by $\lambda_z$ to give AUC from time 0 to infinite time ($AUC_\infty$).

An analysis of variance (ANOVA) was performed for $T_{max}$ and the natural logarithms of $C_{max}$ and AUC. The model included effects for sequence, subject nested within sequence, period and regimen. The effects of sequence, period and regimen were fixed, while the effect of subject was random. For the test on sequence effects, the denominator sum of squares for the F statistic was the sum of squares for subject nested within sequence. For the tests on period and regimen effects, the denominator sum of squares was the residual sum of squares. The statistical tests were performed at a significance level of 0.05.

The bioavailability of the high-fat meal regimen relative to that of the fasting regimens was assessed by the two one-sided tests procedure via 90% confidence intervals.

Mean $\pm$ standard deviation (SD) and the minimum and maximum values observed for the pharmacokinetic parameters of fenofibric acid after administration are listed in the Tables shown in Examples 4, 5, 6 or 7. In the Tables shown in Examples 4, 5, 6 and 7, the top value shown in each cell is the mean $\pm$ SD, unless otherwise noted. The middle value shown in each cell is the minimum. The lower value shown in each cell is the maximum.

**EXAMPLE 4: Food Effect -Formulations 1, 2, 5, 6, 7 and 8**

Formulations 1 and 2, 5 and 6, 7 and 8 were tested in fasting and fed healthy subjects as described in Example 3. Fenofibric acid content in plasma was measured for each sample. The data from these studies are summarized below in Tables 25-27.

**Table 25 Pharmacokinetic Parameters of Fenofibric Acid from Formulations 1 and 2**

| Pharmacokinetic Parameters (units) | | Regimens£ | | | | |
|---|---|---|---|---|---|---|
| | | A: Reference, Low-Fat Meal (N = 48) | B: Formulation 1, High-Fat Meal (N = 18) | C: Formulation 1, Fasting (N = 30) | D: Formulation 2, High-Fat Meal (N = 18) | E: Formulation 2, Fasting (N = 30) |
| $T_{max}$ | (h) | $4.8 \pm 2.0$ | $5.9 \pm 1.5$ | $4.5 \pm 2.9$† | $9.6 \pm 3.9$† | $6.1 \pm 3.8$ |
| | | 2.0 | 4.0 | 2.0 | 5.0 | 3.0 |
| | | 12.0 | 9.0 | 18.0 | 18.0 | 24.0 |
| $C_{max}$ | (µg/mL) | $5.89 \pm 1.67$ | $7.62 \pm 1.32$§ | $3.92 \pm 1.55$*† | $5.03 \pm 1.74$† | $2.96 \pm 1.59$* |
| | | 3.23 | 5.43 | 1.90 | 2.51 | 0.86 |
| | | 11.26 | 11.27 | 8.72 | 8.72 | 7.59 |
| $AUC_{\infty}$$ | (µg•h/mL) | $112.2 \pm 41.3$ | $111.3 \pm 31.4$§ | $99.6 \pm 37.4$*† | $97.7 \pm 40.3$† | $71.7 \pm 43.3$* |
| | | 51.3 | 60.6 | 31.2 | 40.6 | 23.7 |
| | | 231.5 | 175.3 | 194.0 | 187.7 | 204.8 |
| $t_{1/2}$¢ | (h) | 15.9 | 14.4 | 14.8* | 14.4 | 15.1 |
| | | 9.4 | 8.4 | 9.5 | 8.8 | 8.4 |
| | | 29.4 | 22.5 | 26.3 | 31.9 | 26.1 |

£ Regimens B, C, D and E were administered as a 130 mg fenofibric acid capsule. Regimen A was administered as a 200 mg fenofibrate capsule.
\* Statistically significantly different from Regimen A (ANOVA, $p < 0.05$).
§ Statistically significantly different from Regimen C (ANOVA, $p < 0.05$).
† Statistically significantly different from Regimen E (ANOVA, $p < 0.05$).
¢ Harmonic mean, evaluations of $t_{1/2}$ were based on statistical tests for $\lambda z$.

**Table 26 Pharmacokinetic Parameters of Fenofibric Acid from <u>Formulations</u> 5 and 6**

| Pharmacokinetic Parameters (units) | | Regimens£ | | | | |
|---|---|---|---|---|---|---|
| | | A: Reference, Low-Fat Meal (N=41) | B: Formulation 5, High-Fat Meal (N = 21) | C: Formulation 5, Fasting (N=21) | D: Formulation 6, High-Fat Meal (N = 21) | E: Formulation 6, Fasting (N = 21) |
| $T_{max}$ | (h) | $4\text{-}6 \pm 1.3$ | $12.8 \pm 4.5$*§ | $5.0 \pm 2.1$ | $18.5 \pm 14.2$*† | $7.9 \pm 3.3$ |
| | | 3.0 | 7.0 | 3.0 | 5.0 | 4.0 |
| | | 10.0 | 24.0 | 10.0 | 72.0 | 18.0 |
| $C_{max}$ | (µg/mL) | $8.53 \pm 2.27$ | $5.51 \pm 1.54$*§ | $6.40 \pm 0.93$* | $4.04 \pm 2.49$* | $4.03 \pm 1.46$* |
| | | 3.19 | 3.12 | 4.60 | 0.89 | 1.96 |
| | | 12.48 | 9.78 | 8.01 | 10.68 | 8.43 |
| $AUC_t$ | (µg•h/mL) | $154.4 \pm 43.2$ | $136.1 \pm 34.2$* | $143.5 \pm 41.1$ | $106.9 \pm 52.3$* | $108.4 \pm 34.0$* |
| | | 77.5 | 70.2 | 77.9 | 38.8 | 64.6 |
| | | 243.8 | 205.6 | 224.7 | 231.1 | 170.6 |
| $AUC_{\infty}$$ | (µg•h/mL) | $159.6 \pm 46.7$ | $140.3 \pm 35.6$* | $148.4 \pm 44.5$ | $115.2 \pm 56.9$* | $110.4 \pm 34.9$* |
| | | 79.5 | 70.8 | 78.8 | 51.4 | 65.4 |
| | | 257.9 | 212.0 | 238.4 | 239.9 | 174.2 |

(continued)

| Pharmacokinetic Parameters (units) | | Regimens£ | | | | |
|---|---|---|---|---|---|---|
| | | A: Reference, Low-Fat Meal (N=41) | B: Formulation 5, High-Fat Meal (N = 21) | C: Formulation 5, Fasting (N=21) | D: Formulation 6, High-Fat Meal (N = 21) | E: Formulation 6, Fasting (N = 21) |
| $t_{1/2}$¢$ | (h) | 22.27 ± 6.50 | 20.47 ± 5.98* | 21.34 ± 7.36 | 19.27 ± 5.27* | 18.71 ± 5.14* |
| | | 11.79 | 12.81 | 11.98 | 11.60 | 9.71 |
| | | 47.07 | 35.58 | 34.83 | 69.46 | 25.01 |
| CL/F | (L/h) | NA | 0.99 ± 0.29 | 0.95 ± 0.29 | 1.36 ± 0.54 | 1.29 ± 0.38 |
| | | | 0.61 | 0.55 | 0.54 | 0.75 |
| | | | 1.84 | 1.65 | 2.53 | 1.99 |

£ Regimens B and C were administered as a single capsule containing fenofibric acid choline salt mini-tablets equivalent to 130 mg fenofibric acid. Regimens D and E were administered as a single-unit tablet containing fenofibric acid choline salt equivalent to 130 mg fenofibric acid. Regimen A was administered as a 200 mg fenofibrate capsule.
* Statistically significantly different from Regimen A (ANOVA, $p < 0.05$).
§ Statistically significantly different from Regimen C (ANOVA, $p < 0.05$).
† Statistically significantly different from Regimen E (ANOVA, $p < 0.05$).
¢ Harmonic mean and pseudo-standard deviation. Harmonic mean evaluations of $t_{1/2}$ were based on statistical tests for $\lambda_z$.
$ For Regimen D, N = 20 for $AUC_\infty$ and $t_{1/2}$.

**Table 27 Pharmacokinetic Parameters of Fenofibric Acid from Formulations 7 and 8**

| Pharmacokinetic Parameters (units) | | Regimens£ | | | | |
|---|---|---|---|---|---|---|
| | | A: Reference, Fenofibrate, Low-Fat Meal (N = 39) | B: Formulation 7, High-Fat Meal (N = 19) | C: Formulation 7, Fasting (N=19) | D: Formulation 8, High-Fat Meal (N = 20) | E: Formulation 8, Fasting (N = 18) |
| | | 4.8 ± 0.9 | 9.9 ± 1.4*§ | 4.7 ± 2.0 | 9.7 ± 2.6* | 9.8 ± 3.6* |
| | | 3.0 | 7.0 | 3.0 | 5.0 | 4.0 |
| $T_{max}$ | (h) | 7.0 | 12.0 | 10.0 | 18.0 | 18.0 |
| | | 7.53 ± 2.44 | 4.16 ± 1.28* | 4.89 ± 1.22* | 5.77 ± 2.12*† | 3.68 ± 1.13* |
| | | 1.21 | 2.20 | 3.10 | 1.78 | 1.68 |
| $C_{max}$ | (9$^9$/ML) | 11.64 | 7.31 | 7.70 | 9.77 | 6.02 |
| | | 149.5 ± 47.7 | 122.3 ± 47.3* | 121.2 ± 38.1* | 120.3 ± 38.7* | 114.4 ± 30.5* |
| | | 39.6 | 52.9 | 50.8 | 65.5 | 69.6 |
| $AUC_t$ | (μg•h/mL) | 241.3 | 244.8 | 209.1 | 186.1 | 179.7 |
| | | 156.3 ± 54.2 | 131.8 ± 58.6* | 128.9 ± 50.8* | 123.3 ± 41.7* | 117.6 ± 32.6' |
| | | 41.6 | 53.6 | 51.7 | 66.4 | 70.5 |
| $AUC_\infty$ | (μg•h/mL) | 277.5 | 294.9 | 272.6 | 197.4 | 192.6 |
| | | 20.29 ± 6.50 | 20.18 ± 8.22 | 20.18 ± 8.79 | 18.73 ± 4.27*† | 19.91 ± 4.33 |
| | | 10.70 | 9.80 | 9.96 | 13.33 | 14.18 |
| $t_{1/2}$¢ | (h) | 62.47 | 51.07 | 64.19 | 39.11 | 31.00 |
| | | NA | 1.18 ± 0.53 | 1.15 ± 0.46 | 1.18 ± 0.40 | 1.19 ± 0.33 |
| | | | 0.44 | 0.48 | 0.66 | 0.68 |

(continued)

| Pharmacokinetic Parameters (units) | Regimens£ | | | | |
|---|---|---|---|---|---|
| | A: Reference, Fenofibrate, Low-Fat Meal (N = 39) | B: Formulation 7, High-Fat Meal (N = 19) | C: Formulation 7, Fasting (N=19) | D: Formulation 8, High-Fat Meal (N = 20) | E: Formulation 8, Fasting (N = 18) |
| CL/F (L/h) | | 2.43 | 2.52 | 1.96 | 1.84 |

£ Regimens B and C were administered as one capsule of 130 mg fenofibric acid as granules. Regimens D and E were administered as one capsule of 130 mg fenofibric acid as mini-tablets. Regimen A was administered as a 200 mg fenofibrate capsule.
* Statistically significantly different from Regimen A (ANOVA, $p < 0.05$).
§ Statistically significantly different from Regimen C (ANOVA, $p < 0.05$).
† Statistically significantly different from Regimen E (ANOVA, $p < 0.05$).
¢ Harmonic mean and pseudo-standard deviation. Harmonic mean evaluations of $t_{1/2}$ were based on statistical tests for $\lambda_z$.
NA = Not applicable.

**Example 5- Food Effect of Formulation 10**

Formulation 10 was tested in healthy subjects under fasting and high fat fed conditions. The formulation was utilized in a single dose, open-label, randomized, crossover study as described in Example 3. Fenofibric acid content in plasma was measured for each sample. The data from these studies are summarized below in Table 28.

**Table 28 Pharmacokinetic Parameters of Fenofibric Acid from Formulation 10**

| Regimen | N | Fenofibric Acid Pharmacokinetic Parameters (Units) | | | | |
|---|---|---|---|---|---|---|
| | | $T_{max}$ (h) | $C_{max}$ (µg/mL) | $AUC_t$ (µg•h/mL) | $AUC_\infty$ (µg•h/mL) | $t_{1/2}{}^\dagger$ (h) |
| Formulation 10 (Fed) | 24 | 10.3 ± 4.8<br>5.0<br>24.0 | 6.85 ± 1.91<br>3.299<br>11.225 | 140.4 ± 38.1<br>86.236<br>261.952 | 143.0 ± 40.1<br>86.909<br>272.830 | 18.4<br>11.16<br>30.02 |
| Formulation 10 (fasting) | 24 | 4.3 ± 1.2<br>3.0<br>8.0 | 8.01 ± 2.01<br>5.207<br>13.268 | 137.8 ± 46.1<br>56.173<br>283.220 | 139.9 ± 47.6<br>56.938<br>291.581 | 18.0<br>12.22<br>28.84 |
| Reference | 24 | 4.6 ± 0.9<br>3.0<br>7.0 | 9.81 ± 2.21<br>6.439<br>13.511 | 159.2 ± 47.5<br>74.933<br>285.132 | 162.3 ± 49.6<br>76.492<br>296.475 | 19.0<br>11.98<br>30.58 |
| † Harmonic mean. | | | | | | |

**Example 6 - Bioequivalence of Formulations 9, 10 and 11**

Formulations 9, 10 and 11 were tested in fasting healthy subjects in a single dose, open-label, randomized, three period, crossover study as described in Example 3. Fenofibric acid content in plasma was measured for each sample. The data from these studies are summarized below in Table 29.

**Table 29 Pharmacokinetic Parameters of Fenofibric Acid from Formulations 9,10 and 11**

| Regimen | N | Fenofibric Acid Pharmacokinetic Parameters (units) | | | | |
|---|---|---|---|---|---|---|
| | | $T_{max}$ (h) | $C_{max}$ (µg/mL) | $AUC_t$ (µg•h/mL) | $AUC_\infty$ (µg•h/mL) | $t_{1/2}$ (h) |
| Reference | 40 | 4.7 ± 1.2<br>3.0<br>9.0 | 9.86 ± 2.44<br>3.81<br>15.21 | 163 ± 49<br>89.9<br>308.3 | 166 ± 51<br>90.6<br>315.6 | 19.3<br>12.30<br>35.00 |

(continued)

| Regimen | N | $T_{max}$ (h) | $C_{max}$ (μg/mL) | $AUC_t$ (μg•h/mL) | $AUC_\infty$ (μg•h/mL) | $t_{1/2}$ (h) |
|---|---|---|---|---|---|---|
| | | | **Fenofibric Acid Pharmacokinetic Parameters (units)** | | | |
| Formulation 9 | 40 | $4.6 \pm 1.5$ | $7.56 \pm 1.97$ | $141 \pm 44$ | $143 \pm 45$ | 17.5 |
| | | 3.0 | 4.06 | 71.5 | 71.9 | 12.58 |
| | | 12.0 | 12.10 | 242.8 | 246.8 | 34.48 |
| Formulation 10 | 40 | $4.3 \pm 1.2$ | $8.08 \pm 2.37$ | $142 \pm 45$ | $144 \pm 47$ | 17.9 |
| | | 3.0 | 2.82 | 73.5 | 73.9 | 11.07 |
| | | 9.0 | 14.72 | 249.7 | 254.9 | 35.54 |
| Formulation 11 | 40 | $4.3 \pm 1.3$ | $9.14 \pm 2.78$ | $143 \pm 45$ | $145 \pm 47$ | 17.3 |
| | | 3.0 | 3.84 | 65.6 | 66.3 | 12.27 |
| | | 8.0 | 15.96 | 247.0 | 254.5 | 34.73 |

## Example 7 - Bioequivalence of Formulations 10, 12 and 13

Formulations 10, 12 and 13 were tested in healthy subjects under fasting conditions. Each formulation was utilized in a single dose, open-label, randomized, crossover study as described in Example 3. Fenofibric acid content in plasma was measured for each sample. The data from these studies are summarized below in Table 30. Note that the "reference" product referred to in Table 30 is a conventional capsule containing 135 mg of 2-[4-(4-chlorobenzoyl)phenoxy]-2-methyl-propanoic acid (which has also been referred to herein as "Neat Drug").

**Table 30 - Pharmacokinetic Parameters of Fenofibric Acid from Formulations 10, 12 and 13**

| Regimen | N | $T_{max}$ | $C_{max}$ | $AUC_t$ | $AUC_\infty$ | $t_{1/2}$ |
|---|---|---|---|---|---|---|
| | | | **Fenofibric Acid Pharmacokinetic Parameters (units)** | | | |
| Neat Drug | 24 | $2.6 \pm 0.9$ | $11.24 \pm 2.24$ | $166.0 \pm 57.3$ | $168.9 \pm 59.7$ | 20.4 |
| | | 1.5 | 8.16 | 95.64 | 95.98 | 13.27 |
| | | 4.0 | 16.86 | 326.32 | 332.59 | 28.19 |
| Formulation 12 | 24 | $4.1 \pm 1.6$ | $8.70 \pm 2.38$ | $156.2 \pm 47.6$ | $159.5 \pm 50.2$ | 20.5 |
| | | 2.0 | 4.29 | 98.29 | 98.29 | 13.67 |
| | | 10.0 | 13.59 | 297.56 | 297.56 | 28.46 |
| Formulation 10 | 24 | $3.9 \pm 0.8$ | $8.01 \pm 1.59$ | $161.5 \pm 50.1$ | $165.6 \pm 53.9$ | 20.5 |
| | | 2.0 | 4.81 | 90.19 | 91.19 | 13.85 |
| | | 5.0 | 11.26 | 271.94 | 289.99 | 31.78 |
| Formulation 13 | 24 | $6.0 \pm 2.5$ | $5.59 \pm 1.31$ | $147.9 \pm 45.5$ | $152.0 \pm 48.2$ | 21.5 |
| | | 3.0 | 2.84 | 78.54 | 79.14 | 14.12 |
| | | 12.0 | 7.99 | 238.46 | 249.80 | 35.65 |

## EXAMPLE 8 - Summary of Pharmacokinetic Parameters

Table 31 below provides a summarized point estimate of $C_{max}$ and AUC for Formulations 1, 2, 5, 6, 7, 8, 9, 10, 11, 12 and 13 for the studies described in Examples 4-7.

**Table 31 Summary of Pharmacokinetic Parameters of Fenofibric Acid from Formulations 1-2, 5-13, relative to the Reference**

| | Point estimate of $C_{max}$ of test formulation (fasting) relative to reference | Point estimate of $C_{max}$ of test formulation (fed) relative to reference | Point estimate of $AUC_\infty$ (fasting) relative to reference | Point estimate of $AUC_\infty$ (fed) relative to reference |
|---|---|---|---|---|
| Formulation 1 | 0.63 | 1.40 | 0.85 | 1.04 |
| Formulation 2 | 0.44 | 0.86 | 0.58 | 0.91 |

(continued)

|  | Point estimate of $C_{max}$ of test formulation (fasting) relative to reference | Point estimate of $C_{max}$ of test formulation (fed) relative to reference | Point estimate of $AUC_{\infty}$ (fasting) relative to reference | Point estimate of $AUC_{\infty}$ (fed) relative to reference |
|---|---|---|---|---|
| Formulation 5 | 0.83 | 0.70 | 0.95 | 0.90 |
| Formulation 6 | 0.43 | 0.44 | 0.67 | 0.68 |
| Formulation 7 | 0.66 | 0.56 | 0.761 | 0.761 |
| Formulation 8 | 0.52 | 0.79 | 0.868 | 0.86 |
| Formulation 9 | 0.765 | | 0.859 | |
| Formulation 10 (Example 6) | 0.808 | | 0.862 | |
| Formulation 10 (Example 7) | 0.814 | 0.69 | 0.857 | 0.89 |
| Formulation 11 | 0.911 | | 0.868 | |
| Formulation 12 | N/A | N/A | N/A | N/A |
| Formulation 13 | N/A | N/A | N/A | N/A |
| N/A: Not tested against the 200 mg fenofibrate capsules. +Antilogarithm of the difference (test minus reference) of the least squares means for logarithms. * AUC values equivalent to the 200 mg fenofibrate reference. | | | | |

## Example 9 - Osmotic Pump Formulation

This example describes how to make an osmotic pump formulation comprising fenofibric acid choline salt. The size of each tablet is approximately 2 mm in diameter. The formulation contains the ingredients listed in Table 32 below.

**Table 32**

| Ingredient | % W/W (based on core tablet weight) |
|---|---|
| Core | |
| Intra-granular | |
| 2-[4-(4-chlorobenzoyl)phenoxy]-2-methyl-propanoic acid choline salt | 12.0% |
| Dicalcium Phosphate, anhydrous | 15.0% |
| Avicel PH101 | 24.0% |
| PVP 30 | 5.0% |
| Lactose Monohydrate | 43.0% |
| Extra-granular | |
| Magnesium stearate | 1.0% |
| Coating | |
| Opadry Clear | 2.0% |

(continued)

| Ingredient | % W/W (based on core tablet weight) |
|---|---|
| Eudragit® NE 30D and Talc | ~26.6% |
| A hole was created using a push pin on the above coated tablets. | |

Manufacturing processes of the above-described osmotic pump formulation is as follows:

| 1 | Charge intra-granular ingredients into a high shear granulator and dry mix until uniform |
|---|---|
| 2 | Add appropriate amount of water into the above high shear granulator and start granulation. |
| 3 | Stop water addition and continue granulation for an appropriate amount of time. |
| 4 | Discharge the above granulation through a mesh screen and place in an over for drying. |
| 5 | Pass the dry granulation through a mesh screen. Mill granulation retained on the screen. |
| 6 | Screen magnesium stearate through a mesh screen and charge into the above blender. Blend for an appropriate amount of time. |
| 7 | Discharge the above powder blend into a rotary press machine. Compress tablets into the target tablet weight. |
| 8 | Charge water, talc, and Eudragit® a container and mix. |
| 9 | Charge above compressed tablets into a Wurster coater and start coating with Opadry Clear to the target weight. Continue coating with Eudragit® to the target weight. |
| 10 | Drill a hole using a push pin on each coated tablet. |

Dissolution values (the dissolution values were determined by taking the mean of two (2) replicates) of the above-described osmotic pump formulation obtained above from a single pH dissolution medium at stirring speed of 100 RPM as shown below in Table 33. As can be seen in Table 33, the dissolution rate (% release) from the prototype osmotic pump would not be expected to achieve the *in vivo* properties suitable for a therapeutically effective solid dosage form. Nonetheless, one skilled in the art would recognize that modifications and/or optimizations could be utilized to improve the dissolution rate of the osmotic pump, including, but not limited to, increasing the area of holes (namely, one or both of increasing the diameter encompassed by the holes or the number of holes) or modifying the thickness of one or both of the enteric coating and rate-controlling mechanism.

**Table 33**

| Time (hr) | 0 | 1 | 2 | 4 | 6 | 8 | 12 | 16 |
|---|---|---|---|---|---|---|---|---|
| % Release | 0 | 11.6 | 6.6 | 15 | 26.1 | 38.6 | 62.5 | 80.7 |

**Example 10 - Dissolution Values for Formulations 3 and 4**

Dissolution values for Formulations 3 and 4 were determined by taking the mean of six (6) replicates from a single pH dissolution medium at stirring speed of 75 RPM (for Formulation 3) or 100 RPM (for Formulation 4) as shown below in Table 34.

**Table 34**

| Time (hr) | 0 | 1 | 2 | 4 | 6 | 8 | 12 | 16 | 20 |
|---|---|---|---|---|---|---|---|---|---|
| Form 3 | 0 | 18.4 | 31.1 | 52.4 | 68.8 | 80.9 | 97.2 | 105.3 | 107.2 |
| Form 4 | 0 | 64.0 | 79.1 | 90.6 | 95.3 | 98.2 | 101.8 | 103.8 | 105.1 |

One skilled in the art would readily appreciate that the present invention is well adapted to carry out the objects and obtain the ends and advantages mentioned, as well as those inherent therein. The compositions, formulations, methods, procedures, treatments, molecules, specific compounds described herein are presently representative of preferred embodiments, are exemplary, and are not intended as limitations on the scope of the invention. It will be readily apparent to one skilled in the art that varying substitutions and modifications may be made to the invention disclosed herein without departing from the scope and spirit of the invention.

All patents and publications mentioned in the specification are indicative of the levels of those skilled in the art to which the invention pertains.

The invention illustratively described herein suitably may be practiced in the absence of any element or elements, limitation or limitations which is not specifically disclosed herein. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

**Claims**

1.  An oral pharmaceutical formulation comprising at least one active agent, wherein the active agent is a salt of 2-[4-(4-chlorobenzoyl)phenoxy]-2-methyl-propanoic acid with choline, and wherein the formulation further comprises at least one enteric coating.

2.  The formulation of claim 1, wherein the formulation is a modified release oral pharmaceutical formulation.

3.  The formulation of claim 2, further comprising at least one rate-controlling mechanism.

4.  The formulation of claim 2 or claim 3, wherein the at least one enteric coating is selected from the group consisting of: one or more methacrylic acid copolymers, ethyl acrylate/methacrylic acid copolymers, cellulose acetate phthalate, cellulose acetate butyrate, hydroxypropyl methylcellulose phthalate, hydroxypropyl methylcellulose acetate succinate, polyvinyl acetate phthalate, cellulose acetate trimellitate, carboxymethylethylcellulose or shellac.

5.  The formulation of claim 3, wherein at least one rate-controlling mechanism is a hydrophilic agent, hydrophobic agent or combinations thereof.

6.  The formulation of claim 5, wherein the hydrophilic agent is a cellulose, polyethylene oxide, polyethylene glycol, xanthan gum, alginates, polyvinyl pyrrolidone, starch, cross-linked homopolymers or copolymers of acrylic acid.

7.  Combination therapy formulation comprising an oral pharmaceutical formulation of any of claims 1 to 6 in the form of a solid dosage formulation, and, within the same solid dosage formulation as the said formulation according to any of claims 1 to 6, or located in a separate and distinct dosage form for co-administration along with the said formulation according to any of claims 1 to 6, an anti-hypertensive agent, preferably selected from the group consisting of: amlodipine, benazepril, benidipine, candesartan, captopril, carvedilol, darodipine, dilitazem, diazoxide, doxazosin, enalapril, epleronone, eprosartan, felodipine, fenoldopam, fosinopril, guanabenz, iloprost, irbesartan, isradipine, lercardinipine, lisinopril, losartan, minoxidil, nebivolol, nicardipine, nifedipine, nimodipine, nisoldipine, omapatrilat, phenoxybenzamine, prazosin, quinapril, reserpine, semotiadil, sitaxsentan, terazosin, telmisartan, labetolol, triamterene, metoprolol, methyldopa, ramipril, olmesartan, verapamil, clonidine, bendromethiazide, torsemide, hydrochlorothiazide, spinronolactone, perindopril, hydralazine, betaxolol, furosimide, acebutolol, atenolol, bisoprolol, nadolol, penbutolol, pindolol, propranolol, timolol, indapamide, trandolopril, amiloride, moexipril, metolozone, or valsartan.

8.  Combination therapy formulation comprising an oral pharmaceutical formulation of any of claims 1 to 6 in the form of a solid dosage formulation, and, within the same solid dosage formulation as the said formulation according to any of claims 1 to 6, or located in a separate and distinct dosage form for co-administration along with the said formulation according to any of claims 1 to 6, a lipid regulating agent, preferably selected from the group consisting of: atorvastatin, simvastatin, fluvastatin, pravastatin, lovastatin, cerivastatin, rosuvastatin, pitavastatin, clofibric acid, niacin/nicotinic acid, torcetrapib, colestipol, omega-3 acid ethyl esters, colesevelam, cholestyramine, ezetimibe, MD-0727, gemfibrozil or probucol.

**Patentansprüche**

1.  Orale pharmazeutische Formulierung, umfassend wenigstens einen aktiven Wirkstoff, wobei der aktive Wirkstoff ein Salz aus 2-[4-(4-Chlorbenzoyl)Phenoxy]-2-Methyl-Propansäure mit Cholin ist und wobei die Formulierung ferner wenigstens eine magensaftresistente Beschichtung umfasst.

2.  Formulierung von Anspruch 1, wobei die Formulierung eine orale pharmazeutische Formulierung mit modifizierter Freisetzung ist.

3.  Formulierung von Anspruch 2, ferner umfassend wenigstens einen Mechanismus, der die Freisetzungsgeschwindigkeit reguliert.

4.  Formulierung von Anspruch 2 oder Anspruch 3, wobei die wenigstens eine magensaftresistente Beschichtung aus der aus einem oder mehreren Methacrylsäure-Copolymeren, Ethylacrylat/Methacrylsäure-Copolymeren, Celluloseacetatphthalat, Celluloseacetatbutyrat, Hydroxypropylmethylcellulosephthalat, Hydroxypropylmethylcelluloseacetatsuccinat, Polyvinylacetatphthalat, Celluloseacetattrimellitat, Carboxymethylethylcellulose oder Schellack bestehenden Gruppe gewählt ist.

5.  Formulierung von Anspruch 3, wobei wenigstens ein Mechanismus, der die Freisetzungsgeschwindigkeit reguliert, ein hydrophiler Wirkstoff, ein hydrophober Wirkstoff oder eine Kombination davon ist.

6.  Formulierung von Anspruch 5, wobei der hydrophile Wirkstoff eine Cellulose, Polyethylenoxid, Polyethylenglycol, Xanthangummi, Alginate, Polyvinylpyrrolidon, Stärke, vernetzte Homopolymere oder Copolymere aus Acrylsäure ist.

7.  Formulierung für eine Kombinationstherapie, umfassend eine orale pharmazeutische Formulierung von einem der Ansprüche 1 bis 6 in Form einer festen Darreichungsformulierung und, innerhalb derselben festen Darreichungsformulierung wie die Formulierung nach einem der Ansprüche 1 bis 6 oder in einer eigenen und unterschiedlichen Darreichungsform zur gleichzeitigen Gabe zusammen mit der Formulierung nach einem der Ansprüche 1 bis 6, einen blutdrucksenkenden Wirkstoff, der vorzugsweise aus der aus Amlodipin, Benazepril, Benidipin, Candesartan, Captopril, Carvedilol, Darodipin, Diltiazem, Diazoxid, Doxazosin, Enalapril, Eplerenon, Eprosartan, Felodipin, Fenoldopam, Fosinopril, Guanabenz, Iloprost, Irbesartan, Isradipin, Lercanidipin, Lisinopril, Losartan, Minoxidil, Nebivolol, Nicardipin, Nifedipin, Nimodipin, Nisoldipin, Omapatrilat, Phenoxybenzamin, Prazosin, Quinapril, Reserpin, Semotiadil, Sitaxentan, Terazosin, Telmisartan, Labetalol, Triamteren, Metoprolol, Methyldopa, Ramipril, Olmesartan, Verapamil, Clonidin, Bendromethiazid, Torasemid, Hydrochlorothiazid, Spironolacton, Perindopril, Hydralazin, Betaxolol, Furosemid, Acebutolol, Atenolol, Bisoprolol, Nadolol, Penbutolol, Pindolol, Propanolol, Timolol, Indapamid, Trandolapril, Amilorid, Moexipril, Metolazon oder Valsartan bestehenden Gruppe gewählt ist.

8.  Formulierung für eine Kombinationstherapie, umfassend eine orale pharmazeutische Formulierung von einem der Ansprüche 1 bis 6 in Form einer festen Darreichungsformulierung und, innerhalb derselben festen Darreichungsformulierung wie die Formulierung nach einem der Ansprüche 1 bis 6 oder in einer eigenen und unterschiedlichen Darreichungsform zur gleichzeitigen Gabe zusammen mit der Formulierung nach einem der Ansprüche 1 bis 6, einen lipidsenkenden Wirkstoff, der vorzugsweise aus der aus Atorvastatin, Simvastatin, Fluvastatin, Pravastatin, Lovastatin, Cerivastatin, Rosuvastatin, Pitavastatin, Clofibrinsäure, Niacin/Nikotinsäure, Torcetrapib, Colestipol, Omega-3-Säure-Ethylestern, Colesevelam, Colestyramin, Ezetimib, MD-0727, Gemfibrozil oder Probucol bestehenden Gruppe gewählt ist.

**Revendications**

1.  Formulation pharmaceutique orale comprenant au moins un agent actif, dans laquelle l'agent actif est un sel d'acide 2-[4-(4-chloro-benzoyl)phénoxy]-2-méthyl-propanoïque avec de la choline, et dans laquelle la formulation comprend de plus au moins un revêtement entérique.

2.  Formulation selon la revendication 1, dans laquelle la formulation est une formulation pharmaceutique orale à libération modifiée.

3.  Formulation selon la revendication 2, comprenant de plus au moins un mécanisme de contrôle de vitesse.

4. Formulation selon la revendication 2 ou la revendication 3, dans laquelle le au moins un revêtement entérique est choisi dans le groupe constitué de : un ou plusieurs copolymères d'acide méthacrylique, copolymères d'acrylate d'éthyle/acide méthacrylique, acétate phtalate de cellulose, acétate butyrate de cellulose, phtalate d'hydroxypropylméthyl-cellulose, acétate succinate d'hydroxypropylméthylcellulose, poly(acétate phtalate de vinyle), trimellitate acétate de cellulose, carboxyméthyl-éthylcellulose ou shellac.

5. Formulation selon la revendication 3, dans laquelle au moins un mécanisme de contrôle de vitesse est un agent hydrophile, un agent hydrophobe ou des combinaisons de ceux-ci.

6. Formulation selon la revendication 5, dans laquelle l'agent hydrophile est une cellulose, du poly(oxyde d'éthylène), du polyéthylène glycol, de la gomme xanthane, des alginates, de la polyvinylpyrrolidone, de l'amidon, des homopolymères ou copolymèrers réticulés d'acide acrylique.

7. Formulation de thérapie de combinaison comprenant une formulation pharmaceutique orale selon l'une quelconque des revendications 1 à 6 dans la forme d'une formulation de dosage solide, et, dans la même formulation de dosage solide que ladite formulation selon l'une quelconque des revendications 1 à 6, ou disposée dans une forme de dosage séparée et distincte pour une co-administration avec ladite formulation selon l'une quelconque des revendications 1 à 6, un agent antihypertenseur, de préférence choisi dans le groupe constitué par : amlodipine, bénazépril, bénidipine, candésartan, captopril, carvédilol, darodipine, diltiazem, diazoxyde, doxazosine, énalapril, épléronone, éprosartan, félodipine, fénoldopam, fosinopril, guanabenz, iloprost, irbésartan, isradipine, lercardiniprine, lisinopril, losartan, minoxidil, nébivolol, nicardipine, nifédipine, nimodipine, nisoldipine, omapatrilat, phénoxybenzamine, prazosin, quinapril, réserpine, sémotiadil, sitaxsentan, térazosine, telmisartan, labétolol, triamtérène, métoprolol, méthyldopa, ramipril, olmésartan, vérapamil, clonidine, bendrométhiazide, torsémide, hydrochlorothiazide, spinronolactone, périndopril, hydralazine, bétaxolol, furosimide, acébutolol, aténolol, bisoprolol, nadolol, penbutolol, pindolol, propranolol, timolol, indapamide, trandolopril, amiloride, moexipril, métolozone, ou valsartan.

8. Formulation de thérapie de combinaison comprenant une formulation pharmaceutique orale selon l'une quelconque des revendications 1 à 6 dans la forme d'une formulation de dosage solide, et, dans la même formulation de dosage solide que ladite formulation selon l'une quelconque des revendications 1 à 6, ou disposée dans une forme de dosage séparée et distincte pour une co-administration avec ladite formulation selon l'une quelconque des revendications 1 à 6, un agent de régulation des lipides, de préférence choisi dans le groupe constitué par : atorvastatine, simvastatine, fluvastatine, pravastatine, lovastatine, cerivastatine, rosuvastatine, pitavastatine, acide clofibrique, niacine/acide nicotinique, torcetrapib, colestipol, esters éthyliques d'acide oméga-3, colesevelam, cholestyramine, ézétimibe, MD-0727, gemfibrozil ou probucol.

# Figure 1

Figure 2

# Figure 3

EP 2 074 992 B1

# Figure 4

EP 2 074 992 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3907792 A
- US 4895726 A
- US 6074670 A
- US 6277405 B
- US 20050148594 A
- WO 2004054568 A
- US 5286497 A
- US 5737320 A
- US 4088864 A
- US 4200098 A
- US 5573776 A
- US 3173876 A
- US 3276586 A
- US 3541005 A
- US 3541006 A
- US 3546142 A
- US 3133132 A
- US 4160020 A
- US 3845770 A
- US 3916899 A
- US 2799241 A

### Non-patent literature cited in the description

- Guidance for Industry SUPAC-MR: Modified Release Solid Oral Dosage Forms, Scale-Up and Postapproval Changes: Chemistry, Manufacturing, and Controls. In Vitro Dissolution, Testing and In Vivo Bioequivalence Documentation. Center for Drug Evaluation and Research, September 1997, vol. CMC 8, 34
- Remington: The Science and Practice of Pharmacy. Mack Publishing Company, 1995
- Guidance for Industry SUPAC-MR: Modified Release Solid Oral Dosage Forms, Scale-Up and Postapproval Changes: Chemistry, Manufacturing, and Controls. In Vitro Dissolution, Testing and In Vivo Bioequivalence Documentation. Center for Drug Evaluation and Research, September 1997, vol. CMC 8, 32-33
- **T. HIGUCHI.** *J. Pharm. Sci.,* 1963, vol. 52, 1145
- **PEPPAS et al.** *Pharm. Acta. Helv.,* 1985, vol. 60, 110-111
- **J.L. FORD et al.** *Int. J. Pharm.,* 1991, vol. 71, 95-104
- Handbook of Pharmaceutical Controlled Release Technology. Marcel Dekker, Inc, 2000
- Treatise on Controlled Drug Delivery, Fundamentals, Optimization, and Applications. Marcel Dekker, Inc, 1992
- Aqueous polymeric coatings for pharmaceutical compositions. Marcel Dekker, Inc, 1997
- Pharmaceutical compositions: Tablets. Marcel Dekker, Inc, 1990, vol. 3, 77-287
- *J. Am. Pharm. Assoc.,* 1979, vol. 48, 451-459
- Modern Plastics Encyclopedia. 1969, vol. 46, 62-70
- **REMINGTON.** Pharmaceutical Sciences. Mack Publishing Company, 1970, 1626-1678